# EUROPEAN PATENT SPECIFICATION

(11) **EP 2 094 866 B1**
(45) Date of publication and mention of the grant of the patent: **03.07.2013**
(21) Application number: 07864114.9
(22) Date of filing: 08.11.2007
(51) Int. Cl.: C12Q 1/68, A61K 31/41

(54) **DIAGNOSIS AND TREATMENT OF BREAST CANCER**
DIAGNOSE UND BEHANDLUNG VON BRUSTKREBS
DIAGNOSTIC ET TRAITEMENT DU CANCER DU SEIN

(30) Priority: 09.11.2006 US 857930 P
(43) Date of publication of application: 02.09.2009
(73) Proprietor: The Regents of the University of Michigan, Ann Arbor, MI 48109-1280 (US)
(72) Inventor: CHINNAIYAN, Arul M., Plymouth, MI 48170 (US); RHODES, Daniel R., Ann Arbor, MI 48105 (US); TOMLINS, Scott A., Ann Arbor, MI 48105 (US); MEHRA, Rohit, Ann Arbor, MI 48105 (US); VARAMBALLY, Sooryanarayana, Ann Arbor, MI 48105 (US)
(74) Representative: Glawe, Delfs, Moll
(86) International application number: PCT/US2007/084089
(87) International publication number: WO 2008/060945

(56) References cited:
- EP-A1- 1 444 988
- WO-A2-2006/089091
- US-A1- 2003 099 974
- SUGANUMA TAKAYASU ET AL: "Functional expression of the angiotensin II type 1 receptor in human ovarian carcinoma cells and its blockade therapy resulting in suppression of tumor invasion, angiogenesis, and peritoneal dissemination." CLINICAL CANCER RESEARCH : AN OFFICIAL JOURNAL OF THE AMERICAN ASSOCIATION FOR CANCER RESEARCH 1 APR 2005 LNKD- PUBMED:15814650, vol. 11, no. 7, 1 April 2005 (2005-04-01), pages 2686-2694, XP002592232 ISSN: 1078-0432
- TYBITANCLOVA ET AL: "AT1 receptor and ACE mRNA are increased in chemically induced carcinoma of rat mammary gland" MOLECULAR AND CELLULAR ENDOCRINOLOGY, ELSEVIER IRELAND LTD, IE LNKD- DOI:10.1016/J.MCE.2005.01.015, vol. 244, no. 1-2, 1 December 2005 (2005-12-01), pages 42-46, XP005170318 ISSN: 0303-7207
- OGEDEGBE A J ET AL: "ANGIOTENSIN II TYPE 1 (ATI) RECEPTOR EXPRESSION MAY HAVE PROGNOSTIC SIGNIFICANCE IN BREAST CANCER?" JOURNAL OF ENDOCRINOLOGY, SOCIETY FOR ENDOCRINOLOGY, GB, vol. 156, no. SUPPL, 23 March 1998 (1998-03-23), page P19, XP009023086 ISSN: 0022-0795
- PUDDEFOOT J R ET AL: "The role of angiotensin II in the regulation of breast cancer cell adhesion and invasion" ENDOCRINE-RELATED CANCER, vol. 13, no. 3, September 2006 (2006-09), pages 895-903, XP002592233 ISSN: 1351-0088
- LAWRENCE JULIA A ET AL: "A high-risk lesion for invasive breast cancer, ductal carcinoma in situ, exhibits frequent overexpression of retinoid X receptor" CANCER EPIDEMIOLOGY BIOMARKERS AND PREVENTION, vol. 7, no. 1, January 1998 (1998-01), pages 29-35, XP002592234 ISSN: 1055-9965
- LIM KHENG TIAN ET AL: "Nongenomic oestrogen signalling in oestrogen receptor negative breast cancer cells: a role for the angiotensin II receptor AT1" BREAST CANCER RESEARCH, CURRENT SCIENCE, LONDON, GB, vol. 8, no. 3, 28 June 2006 (2006-06-28), page R33, XP021020728 ISSN: 1465-5411
- RESTA L ET AL: "EXPRESSION OF ANGIOTENSIN II TYPE 1 RECEPTOR (AT1) IN BREAST CANCER" VIRCHOWS ARCHIV, SPRINGER INTERNATIONAL, BERLIN, DE, vol. 439, no. 3, 1 September 2001 (2001-09-01), page 449, XP009023097 ISSN: 0945-6317
- MURAMATSU MICHIKO ET AL: "Antitumor effect of angiotensin II type 1 receptor antagonist (candesartan) on mammary cancer in mouse" JOURNAL OF PHARMACOLOGICAL SCIENCES, JAPANESE PHARMACOLOGICAL SOCIETY, TOKYO, JP, vol. 91, no. Supplement I, 1 March 2003 (2003-03-01), page 165P, XP008092169 ISSN: 1347-8613
- ARAFAT ET AL: "Antihypertensives as Novel Antineoplastics: Angiotensin-I-Converting Enzyme Inhibitors and Angiotensin II Type 1 Receptor Blockers in Pancreatic Ductal Adenocarcinoma" JOURNAL OF THE AMERICAN COLLEGE OF SURGEONS, COLLEGE, CHICAGO, IL, US LNKD- DOI:10.1016/J.JAMCOLLSURG.2007.01.067, vol. 204, no. 5, 1 May 2007 (2007-05-01), pages 996-1005, XP022055426 ISSN: 1072-7515
- RHODES DANIEL R ET AL: "AGTR1 overexpression defines a subset of breast cancer and confers sensitivity to losartan, an AGTR1 antagonist." PROCEEDINGS OF THE NATIONAL ACADEMY OF SCIENCES OF THE UNITED STATES OF AMERICA 23 JUN 2009 LNKD- PUBMED:19487683, vol. 106, no. 25, 23 June 2009 (2009-06-23), pages 10284-10289, XP002592235 ISSN: 1091-6490
- TAHMASEBI ET AL.: 'Localisation of renin-angiotensin system (RAS) components in breast' BRITISH JOURNAL OF CANCER vol. 95, June 2006, pages 67 - 74, XP008109015
- STEIN ET AL.: 'Involution of the mouse mammary gland is associated with an immune cascade and an acute-phase response, involving LBP,CD14 and STAT3' BREAST CANCER RESEARCH vol. 6, 2004, pages R75 - R91, XP021012017

## Description

### FIELD OF THE INVENTION

The present invention relates to methods for cancer diagnosis and therapy, including but not limited to, cancer markers. In particular, the present invention relates to AGTR1 markers for breast cancer.

### BACKGROUND OF THE INVENTION

Breast cancer is the second most common form of cancer among women in the U.S., and the second leading cause of cancer deaths among women. While the 1980s saw a sharp rise in the number of new cases of breast cancer, that number now appears to have stabilized. The drop in the death rate from breast cancer is probably due to the fact that more women are having mammograms. When detected early, the chances for successful treatment of breast cancer are much improved.

Breast cancer, which is highly treatable by surgery, radiation therapy, chemotherapy, and hormonal therapy, is most often curable when detected in early stages. Mammography is the most important screening modality for the early detection of breast cancer. Breast cancer is classified into a variety of sub-types, but only a few of these affect prognosis or selection of therapy. Patient management following initial suspicion of breast cancer generally includes confirmation of the diagnosis, evaluation of stage of disease, and selection of therapy. Diagnosis may be confirmed by aspiration cytology, core needle biopsy with a stereotactic or ultrasound technique for nonpalpable lesions, or incisional or excisional biopsy. At the time the tumor tissue is surgically removed, part of it is processed for determination of ER and PR levels.

Prognosis and selection of therapy are influenced by the age of the patient, stage of the disease, pathologic characteristics of the primary tumor including the presence of tumor necrosis, estrogen-receptor (ER) and progesterone-receptor (PR) levels in the tumor tissue, HER2 overexpression status and measures of proliferative capacity, as well as by menopausal status and general health. Overweight patients may have a poorer prognosis (Bastarrachea et al., Annals of Internal Medicine, 120: 18 [1994]). Prognosis may also vary by race, with blacks, and to a lesser extent Hispanics, having a poorer prognosis than whites (Elledge et al., Journal of the National Cancer Institute 86: 705 [1994]; Edwards et al., Journal of Clinical Oncology 16: 2693 [1998]).

The three major treatments for breast cancer are surgery, radiation, and drug therapy. No treatment fits every patient, and often two or more are required. The choice is determined by many factors, including the age of the patient and her menopausal status, the type of cancer (*e.g.,* ductal vs. lobular), its stage, whether the tumor is hormone-receptive or not, and its level of invasiveness.

Breast cancer treatments are defined as local or systemic. Surgery and radiation are considered local therapies because they directly treat the tumor, breast, lymph nodes, or other specific regions. Drug treatment is called systemic therapy, because its effects are wide spread. Drug therapies include classic chemotherapy drugs, hormone blocking treatment (e.g., aromatase inhibitors, selective estrogen receptor modulators, and estrogen receptor downregulators), and monoclonal antibody treatment (e.g., against HER2). They may be used separately or, most often, in different combinations.

There is a need for additional treatments, particularly treatments customized to a patient's tumor.

The document "TAHMASEBI ET AL.: "Localisation of renin-angiotensin system (RAS) components in breast", BRITISH JOURNAL OF CANCER, vol. 95, June 2006, pages 67-74" discloses that AT1 receptor expression, as determined by quantitative RT-PCR, in breast carcinoma is much more abundant than in normal tissue. Furthermore, the document "PUDDEFOOT J R ET AL: "The role of angiotensin II in the regulation of breast cancer cell adhesion and invasion", ENDOCRINE-RELATED CANCER, vol. 13, no. 3, September 2006, pages 895-903 ISSN1351-0088" discloses that Losartan is used for studying the effect of inhibiting AGTR1 in breast cancer.

### SUMMARY OF THE INVENTION

The present invention relates to compositions and methods for cancer diagnosis and therapy as defined in the claims.

### DESCRIPTION OF THE FIGURES

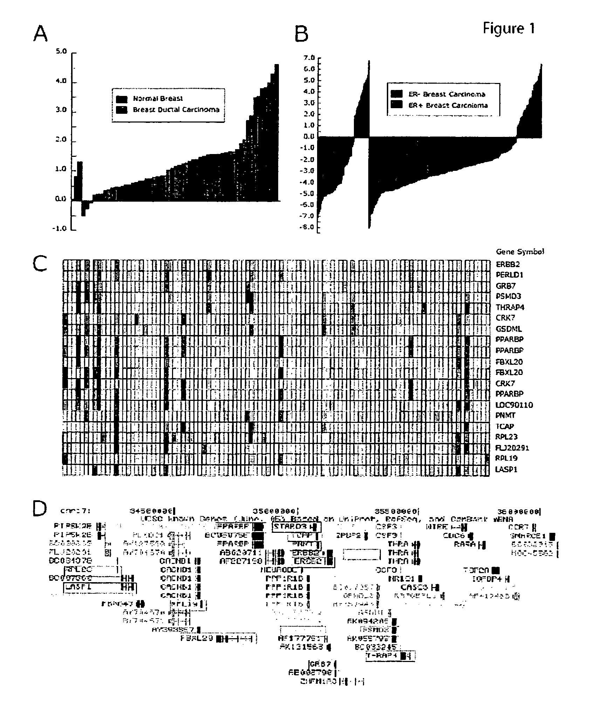

Figure 1 shows Cancer Outlier Profile Analysis (COPA) indicating that ERBB2 exhibits outlier expression in multiple breast cancer microarray datasets. (A) ERBB2 expression profile in the Perou et al. (Nature 406, 747 [2000]) cDNA microarray dataset (n=55). (B) ERBB2 expression profile in the van de Vijver et al. (N Engl J Med 347, 1999 [2002]) oligonucleotide dataset, segregated by estrogen receptor (ER) status (n=295). (C) Co-expression analysis of ERBB2 in breast cancer. (D) UCSC Genome View of ERBB2 and genomic neighbors. Genes significantly co-expressed with ERBB2 in breast cancer are designated with a red box.

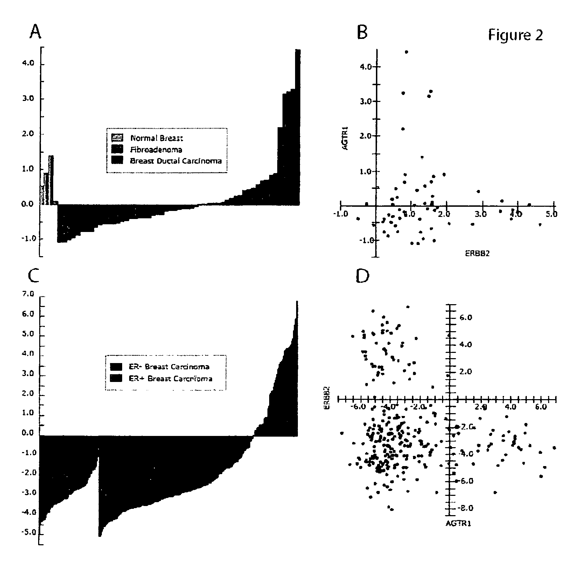

Figure 2 show AGTR1 outlier expression in breast cancer. (A) AGTR1 expression profile in the Perou et al. (Nature 406, 747 [2000]) cDNA microarray dataset (n=55). (B) In the same dataset, AGTR1 expression vs. ERBB2 expression. (C) AGTR1 expression profile in the van de Vijver et al. (N Engl J Med 347, 1999 [2002]) oligonucleotide dataset, segregated by estrogen receptor (ER) status (n=295). (D) AGTR1 expression vs. ERBB2 expression in the same dataset.

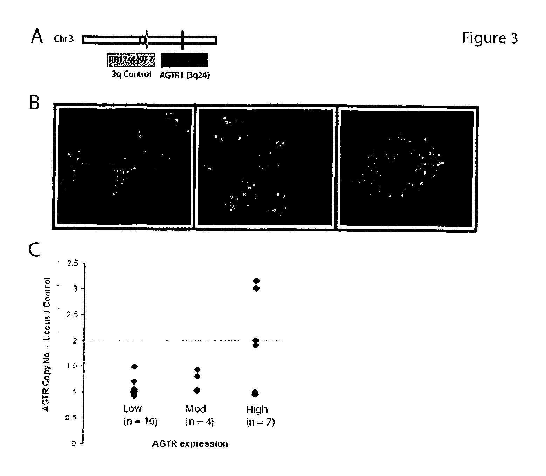

Figure 3 shows copy number analysis of the AGTR1 locus. (A) A schematic of probes used for FISH analysis. (B) Representative image from FISH analysis. The left panel is taken from a representative negative case. (C) Association of AGTR1 over-expression with copy number gain.

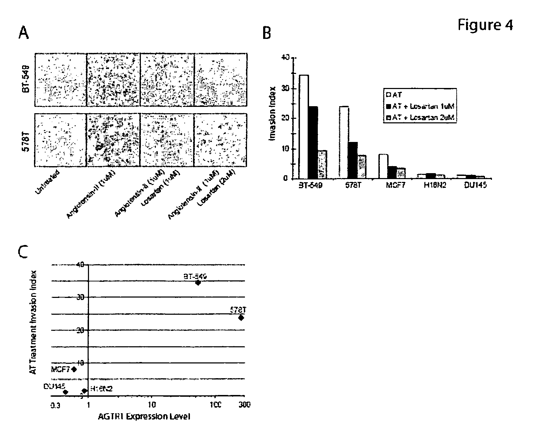

Figure 4 shows cell line analysis of angiotensin II (AT) and losartan effects on invasion and association with AGTR1 expression. (A) Cancer cell line matrigel invasion assays with and without AT and losartan treatment in cancer cell lines with AGTR1 over-expression. (B) Effect of AT and losartan treatment on invasion in 5 cell lines. (C) Association of AGTR1 expression levels, as measured by quantitative RT-PCR, and AT-mediated invasion.

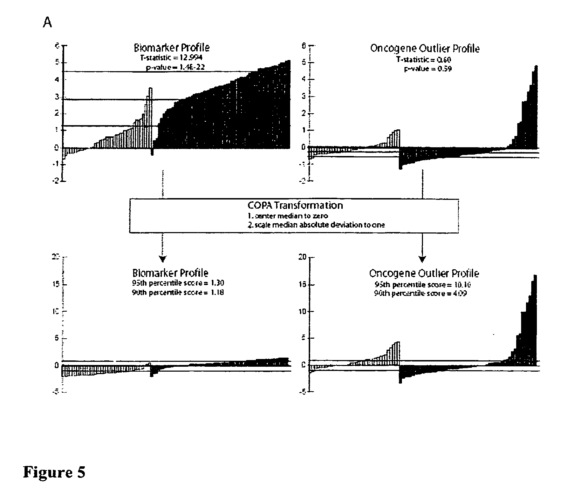

Figure 5 shows Cancer Outlier Profile Analysis (COPA). A. COPA schematic.

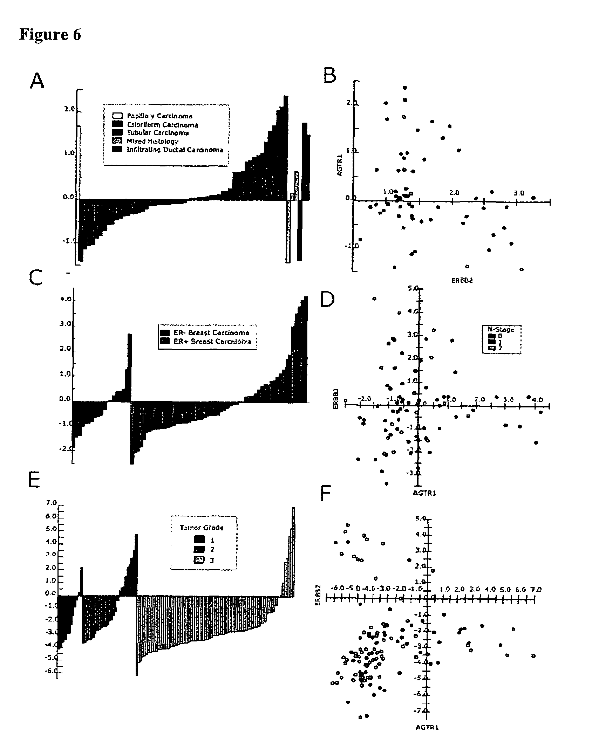

Figure 6 shows AGTR1 expression and comparison with ERBB2 expression in three additional datasets. A, B. Huang et al. (Lancet 361, 1590 [2003]). C, D. Sorlie et al. (Proc Natl Acad Sci U S A 100, 8418 [2003]). E, F. van't Veer et al. (Nature 415, 530 [2002]). Bar graph legends correspond to scatterplots except in panel (D).

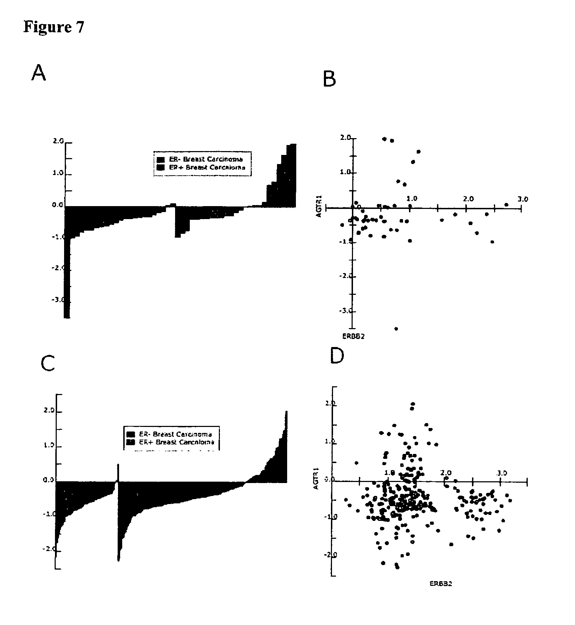

Figure 7 shows AGTR1 expression and comparison with ERBB2 expression in two additional datasets. A, B. West ct al. (Proc Natl Acad Sci U S A 98, 11462 [2001]). C, D. Wang ct al. (Lancet 365, 671 [2005]).

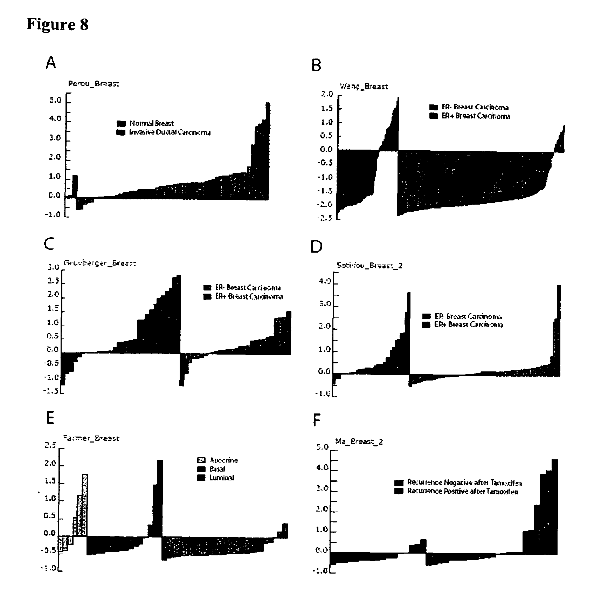

Figure 8 shows LBP outlier expression in breast cancer. (A) LBP expression profile in the Perou et al. (Nature 406, 747 (2000)) dataset. (B) LBP expression profile in the Wang et al. (Lancet 365, 671 (2005)) dataset. (C) LBP expression profile in the Gruvberger et al. (Cancer Res 61, 5979 (2001)) dataset. (D) LBP expression profile in the Sotiriou et al. (Proc Natl Acad Sci U S A 100, 10393 (2003)) dataset. (E) LBP expression profile in the Farmer et al. (Oncogene 24, 4660 (2005)) dataset. (F) LBP expression profile in the Ma et al. (Cancer Cell 5, 607 (2004)) dataset.

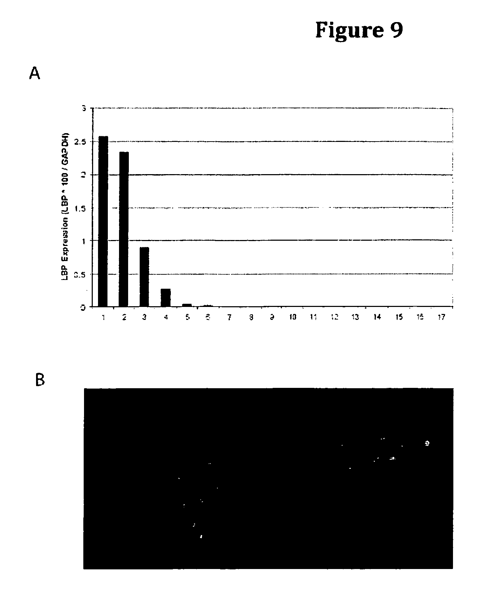

Figure 9 shows validation of LBP expression by RT-PCR and identification of DNA copy number gain by FISH. (A). Expression of LBP across 17 cases. Cases in which no amplification of LBP message occurred in 40 cycles have no bar. Expression is normalized to GAPDH and multiplied by 100. (B). Cases 1 and 2 from A. were evaluated for DNA copy number gain by FISH.

### DEFINITIONS

To facilitate an understanding of the present invention, a number of terms and phrases are defined below:
As used herein, the terms "overexpression of AGTR1" and "overexpression of LBP" refer to a higher level of expression of AGTR1 or LBP nucleic acid (e.g., mRNA or genomic DNA) or protein relative to the level normally found. Expression may be increased at least 10%, preferably at least 20%, even more preferably at least 50%, yet more preferably at least 75%, still more preferably at least 90%, and most preferably at least 100% relative the level of expression normally found (e.g., in non-cancerous tissue). Expression levels may be determined using any suitable method, including, but not limited to, those disclosed herein.

As used herein, the term "post-surgical tissue" refers to tissue that has been removed from a subject during a surgical procedure. Examples include, but are not limited to, biopsy samples, excised organs, and excised portions of organs.

As used herein, the terms "detect", "detecting", or "detection" may describe either the general act of discovering or discerning or the specific observation of a detectably labeled composition.

As used herein, the term "siRNAs" refers to small interfering RNAs. siRNAs may comprise a duplex, or double-stranded region, of about 18-25 nucleotides long; often siRNAs contain from about two to four unpaired nucleotides at the 3' end of each strand. At least one strand of the duplex or double-stranded region of a siRNA is substantially homologous to, or substantially complementary to, a target RNA molecule. The strand complementary to a target RNA molecule is the "antisense strand;" the strand homologous to the target RNA molecule is the "sense strand," and is also complementary to the siRNA antisense strand. siRNAs may also contain additional sequences; examples of such sequences include linking sequences, or loops, as well as stem and other folded structures. siRNAs appear to function as key intermediaries in triggering RNA interference in invertebrates and in vertebrates, and in triggering sequence-specific RNA degradation during posttranscriptional gene silencing in plants.

The term "RNA interference" or "RNAi" refers to the silencing or decreasing of gene expression by siRNAs. It is the process of sequence-specific, post-transcriptional gene silencing in animals and plants, initiated by siRNA that is homologous in its duplex region to the sequence of the silenced gene. The gene may be endogenous or exogenous to the organism, present integrated into a chromosome or present in a transfection vector that is not integrated into the genome. The expression of the gene is either completely or partially inhibited. RNAi may also be considered to inhibit the function of a target RNA; the function of the target RNA may be complete or partial.

As used herein, the term "gene transfer system" refers to any means of delivering a composition comprising a nucleic acid sequence to a cell or tissue. For example, gene transfer systems include, but are not limited to, vectors (*e.g.,* retroviral, adenoviral, adeno-associated viral, and other nucleic acid-based delivery systems), microinjection of naked nucleic acid, polymer-based delivery systems (*e.g.,* liposome-based and metallic particle-based systems), biolistic injection, and the like. As used herein, the term "viral gene transfer system" refers to gene transfer systems comprising viral elements (e.g., intact viruses, modified viruses and viral components such as nucleic acids or proteins) to facilitate delivery of the sample to a desired cell or tissue. As used herein, the term "adenovirus gene transfer system" refers to gene transfer systems comprising intact or altered viruses belonging to the family Adenoviridae.

As used herein, the term "site-specific recombination target sequences" refers to nucleic acid sequences that provide recognition sequences for recombination factors and the location where recombination takes place.

As used herein, the term "nucleic acid molecule" refers to any nucleic acid containing molecule, including but not limited to, DNA or RNA. The term encompasses sequences that include any of the known base analogs of DNA and RNA including, but not limited to, 4-acetylcytosine, 8-hydroxy-N6-methyladenosine, aziridinylcytosine, pseudoisocytosine, 5-(carboxyhydroxylmethyl) uracil, 5-fluorouracil, 5-bromouracil, 5-carboxymethylaminomethyl-2-thiouracil, 5-carboxymethylaminomethyluracil, dihydrouracil, inosine, N6-isopentenyladenine, I-methyladenine, I -methylpseudouracil, 1-methylguanine, 1-methylinosine, 2,2-dimethylguanine, 2-methyladenine, 2-methylguanine, 3-methylcytosine, 5-methylcytosine, N6-methyladenine, 7-methylguanine, 5-methylaminomethyluracil, 5-methoxyaminomethyl-2-thiouracil, beta-D-mannosylqueosine, 5'-methoxycarbonylmethyluracil, 5-methoxyuracil, 2-methylthio-N6-isopentenyladenine, uracil-5-oxyacetic acid methylester, uracil-5-oxyacetic acid, oxybutoxosine, pseudouracil, queosine, 2-thiocytosine, 5-methyl-2-thiouracil, 2-thiouracil, 4-thiouracil, 5-methyluracil, N-uracil-5-oxyacetic acid methylester, uracil-5-oxyacetic acid, pseudouracil, queosine, 2-thiocytosine, and 2,6-diaminopurine.

The term "gene" refers to a nucleic acid (e.g., DNA) sequence that comprises coding sequences necessary for the production of a polypeptide, precursor, or RNA (*e.g.,* rRNA, tRNA). The polypeptide can be encoded by a full length coding sequence or by any portion of the coding sequence so long as the desired activity or functional properties (*e.g.,* enzymatic activity, ligand binding, signal transduction, immunogenicity, etc.) of the full-length or fragment are retained. The term also encompasses the coding region of a structural gene and the sequences located adjacent to the coding region on both the 5' and 3' ends for a distance of about I kb or more on either end such that the gene corresponds to the length of the full-length mRNA. Sequences located 5' of the coding region and present on the mRNA are referred to as 5' non-translated sequences. Sequences located 3' or downstream of the coding region and present on the mRNA are referred to as 3' non-translated sequences. The term "gene" encompasses both cDNA and genomic forms of a gene. A genomic form or clone of a gene contains the coding region interrupted with noncoding sequences termed "introns" or "intervening regions" or "intervening sequences." Introns are segments of a gene that are transcribed into nuclear RNA (hnRNA); introns may contain regulatory elements such as enhancers. Introns are removed or "spliced out" from the nuclear or primary transcript; introns therefore are absent in the messenger RNA (mRNA) transcript. The mRNA functions during translation to specify the sequence or order of amino acids in a nascent polypeptide.

As used herein, the term "heterologous gene" refers to a gene that is not in its natural environment. For example, a heterologous gene includes a gene from one species introduced into another species. A heterologous gene also includes a gene native to an organism that has been altered in some way (*e.g.,* mutated, added in multiple copies, linked to non-native regulatory sequences, etc). Heterologous genes are distinguished from endogenous genes in that the heterologous gene sequences are typically joined to DNA sequences that are not found naturally associated with the gene sequences in the chromosome or are associated with portions of the chromosome not found in nature (*e.g.,* genes expressed in loci where the gene is not normally expressed).

As used herein, the term "oligonucleotide," refers to a short length of single-stranded polynucleotide chain. Oligonucleotides are typically less than 200 residues long (*e.g.,* between 15 and 100), however, as used herein, the term is also intended to encompass longer polynucleotide chains. Oligonucleotides are often referred to by their length. For example a 24 residue oligonucleotide is referred to as a "24-mer". Oligonucleotides can form secondary and tertiary structures by self-hybridizing or by hybridizing to other polynucleotides. Such structures can include, but are not limited to, duplexes, hairpins, cruciforms, bends, and triplexes.

As used herein, the terms "complementary" or "complementarity" are used in reference to polynucleotides (*i.e.,* a sequence of nucleotides) related by the base-pairing rules. For example, the sequence "5'-A-G-T-3'," is complementary to the sequence "3'-T-C-A-5'." Complementarity may be "partial," in which only some of the nucleic acids' bases are matched according to the base pairing rules. Or, there may be "complete" or "total" complementarity between the nucleic acids. The degree of complementarity between nucleic acid strands has significant effects on the efficiency and strength of hybridization between nucleic acid strands. This is of particular importance in amplification reactions, as well as detection methods that depend upon binding between nucleic acids.

The term "homology" refers to a degree of complementarity. There may be partial homology or complete homology (*i.e*., identity). A partially complementary sequence is a nucleic acid molecule that at least partially inhibits a completely complementary nucleic acid molecule from hybridizing to a target nucleic acid is "substantially homologous." The inhibition of hybridization of the completely complementary sequence to the target sequence may be examined using a hybridization assay (Southern or Northern blot, solution hybridization and the like) under conditions of low stringency. A substantially homologous sequence or probe will compete for and inhibit the binding (*i.e.,* the hybridization) of a completely homologous nucleic acid molecule to a target under conditions of low stringency. This is not to say that conditions of low stringency are such that non-specific binding is permitted; low stringency conditions require that the binding of two sequences to one another be a specific (*i.e*., selective) interaction. The absence of non-specific binding may be tested by the use of a second target that is substantially non-complementary (*e.g.,* less than about 30% identity); in the absence of non-specific binding the probe will not hybridize to the second non-complementary target.

When used in reference to a double-stranded nucleic acid sequence such as a cDNA or genomic clone, the term "substantially homologous" refers to any probe that can hybridize to either or both strands of the double-stranded nucleic acid sequence under conditions of low stringency as described above.

A gene may produce multiple RNA species that are generated by differential splicing of the primary RNA transcript. cDNAs that are splice variants of the same gene will contain regions of sequence identity or complete homology (representing the presence of the same exon or portion of the same exon on both cDNAs) and regions of complete non-identity (for example, representing the presence of exon "A" on cDNA I wherein cDNA 2 contains exon "B" instead). Because the two cDNAs contain regions of sequence identity they will both hybridize to a probe derived from the entire gene or portions of the gene containing sequences found on both cDNAs; the two splice variants are therefore substantially homologous to such a probe and to each other.

When used in reference to a single-stranded nucleic acid sequence, the term "substantially homologous" refers to any probe that can hybridize (i.e., it is the complement of) the single-stranded nucleic acid sequence under conditions of low stringency as described above.

As used herein, the term "hybridization" is used in reference to the pairing of complementary nucleic acids. Hybridization and the strength of hybridization (i.e., the strength of the association between the nucleic acids) is impacted by such factors as the degree of complementary between the nucleic acids, stringency of the conditions involved, the Tₘ of the formed hybrid, and the G:C ratio within the nucleic acids. A single molecule that contains pairing of complementary nucleic acids within its structure is said to be "self-hybridized."

As used herein the term "stringency" is used in reference to the conditions of temperature, ionic strength, and the presence of other compounds such as organic solvents, under which nucleic acid hybridizations are conducted. Under "low stringency conditions" a nucleic acid sequence of interest will hybridize to its exact complement, sequences with single base mismatches, closely related sequences (*e.g.,* sequences with 90% or greater homology), and sequences having only partial homology (*e.g*., sequences with 50-90% homology). Under "medium stringency conditions," a nucleic acid sequence of interest will hybridize only to its exact complement, sequences with single base mismatches, and closely relation sequences (*e.g.,* 90% or greater homology). Under "high stringency conditions," a nucleic acid sequence of interest will hybridize only to its exact complement, and (depending on conditions such a temperature) sequences with single base mismatches. In other words, under conditions of high stringency the temperature can be raised so as to exclude hybridization to sequences with single base mismatches.

"High stringency conditions" when used in reference to nucleic acid hybridization comprise conditions equivalent to binding or hybridization at 42°C in a solution consisting of 5X SSPE (43.8 g/l NaCl, 6.9 g/l NaH₂PO₄ H₂O and 1.85 g/l EDTA, pH adjusted to 7.4 with NaOH), 0.5% SDS, 5X Denhardt's reagent and 100 µg/ml denatured salmon sperm DNA followed by washing in a solution comprising 0.1X SSPE, 1.0% SDS at 42°C when a probe of about 500 nucleotides in length is employed.

"Medium stringency conditions" when used in reference to nucleic acid hybridization comprise conditions equivalent to binding or hybridization at 42°C in a solution consisting of 5X SSPE (43.8 g/l NaCl, 6.9 g/l NaH₂PO₄ H₂O and 1.85 g/l EDTA, pH adjusted to 7.4 with NaOH), 0.5% SDS, 5X Denhardt's reagent and 100 µg/ml denatured salmon sperm DNA followed by washing in a solution comprising 1.0X SSPE, 1.0% SDS at 42°C when a probe of about 500 nucleotides in length is employed.

"Low stringency conditions" comprise conditions equivalent to binding or hybridization at 42°C in a solution consisting of 5X SSPE (43.8 g/l NaCl, 6.9 g/l NaH₂PO₄ H₂O and 1.85 g/l EDTA, pH adjusted to 7.4 with NaOH), 0.1% SDS, 5X Denhardt's reagent [50X Denhardt's contains per 500 ml: 5 g Ficoll (Type 400, Pharamcia), 5 g BSA (Fraction V; Sigma)] and 100 µg/ml denatured salmon sperm DNA followed by washing in a solution comprising 5X SSPE, 0.1 % SDS at 42°C when a probe of about 500 nucleotides in length is employed.

The art knows well that numerous equivalent conditions may be employed to comprise low stringency conditions; factors such as the length and nature (DNA, RNA, base composition) of the probe and nature of the target (DNA, RNA, base composition, present in solution or immobilized, etc.) and the concentration of the salts and other components (*e.g.*, the presence or absence of formamide, dextran sulfate, polyethylene glycol) are considered and the hybridization solution may be varied to generate conditions of low stringency hybridization different from, but equivalent to, the above listed conditions. In addition, the art knows conditions that promote hybridization under conditions of high stringency (*e.g*., increasing the temperature of the hybridization and/or wash steps, the use of formamide in the hybridization solution, etc.) (see definition above for "stringency").

As used herein, the term "amplification oligonucleotide" refers to an oligonucleotide that hybridizes to a target nucleic acid, or its complement, and participates in a nucleic acid amplification reaction. An example of an amplification oligonucleotide is a "primer" that hybridizes to a template nucleic acid and contains a 3' OH end that is extended by a polymerase in an amplification process. Another example of an amplification oligonucleotide is an oligonucleotide that is not extended by a polymerase (e.g., because it has a 3' blocked end) but participates in or facilitates amplification. Amplification oligonucleotides may optionally include modified nucleotides or analogs, or additional nucleotides that participate in an amplification reaction but are not complementary to or contained in the target nucleic acid. Amplification oligonucleotides may contain a sequence that is not complementary to the target or template sequence. For example, the 5' region of a primer may include a promoter sequence that is non-complementary to the target nucleic acid (referred to as a "promoter-primer"). Those skilled in the art will understand that an amplification oligonucleotide that functions as a primer may be modified to include a 5' promoter sequence, and thus function as a promoter-primer. Similarly, a promoter-primer may be modified by removal of, or synthesis without, a promoter sequence and still function as a primer. A 3' blocked amplification oligonucleotide may provide a promoter sequence and serve as a template for polymerization (referred to as a "promoter-provider").

As used herein, the term "primer" refers to an oligonucleotide, whether occurring naturally as in a purified restriction digest or produced synthetically, that is capable of acting as a point of initiation of synthesis when placed under conditions in which synthesis of a primer extension product that is complementary to a nucleic acid strand is induced, *(i.e.,* in the presence of nucleotides and an inducing agent such as DNA polymerase and at a suitable temperature and pH). The primer is preferably single stranded for maximum efficiency in amplification, but may alternatively be double stranded. If double stranded, the primer is first treated to separate its strands before being used to prepare extension products. Preferably, the primer is an oligodeoxyribonucleotide. The primer must be sufficiently long to prime the synthesis of extension products in the presence of the inducing agent. The exact lengths of the primers will depend on many factors, including temperature, source of primer and the use of the method.

As used herein, the term "probe" refers to an oligonucleotide (*i.e*., a sequence of nucleotides), whether occurring naturally as in a purified restriction digest or produced synthetically, recombinantly or by PCR amplification, that is capable of hybridizing to at least a portion of another oligonucleotide of interest. A probe may be single-stranded or double-stranded. Probes are useful in the detection, identification and isolation of particular gene sequences. It is contemplated that any probe may be labeled with any "reporter molecule," so that is detectable in any detection system, including, but not limited to enzyme (*e.g.,* ELISA, as well as enzyme-based histochemical assays), fluorescent, radioactive, and luminescent systems.

The term "isolated" when used in relation to a nucleic acid, as in "an isolated oligonucleotide" or "isolated polynucleotide" refers to a nucleic acid sequence that is identified and separated from at least one component or contaminant with which it is ordinarily associated in its natural source. Isolated nucleic acid is such present in a form or setting that is different from that in which it is found in nature. In contrast, non-isolated nucleic acids as nucleic acids such as DNA and RNA found in the state they exist in nature. For example, a given DNA sequence (*e.g.*, a gene) is found on the host cell chromosome in proximity to neighboring genes; RNA sequences, such as a specific mRNA sequence encoding a specific protein, are found in the cell as a mixture with numerous other mRNAs that encode a multitude of proteins. However, isolated nucleic acid encoding a given protein includes, by way of example, such nucleic acid in cells ordinarily expressing the given protein where the nucleic acid is in a chromosomal location different from that of natural cells, or is otherwise flanked by a different nucleic acid sequence than that found in nature. The isolated nucleic acid, oligonucleotide, or polynucleotide may be present in single-stranded or double-stranded form. When an isolated nucleic acid, oligonucleotide or polynucleotide is to be utilized to express a protein, the oligonucleotide or polynucleotide will contain at a minimum the sense or coding strand *(i.e.,* the oligonucleotide or polynucleotide may be single-stranded), but may contain both the sense and anti-sense strands (*i.e.,* the oligonucleotide or polynucleotide may be double-stranded).

As used herein, the term "purified" or "to purify" refers to the removal of components (*e.g*., contaminants) from a sample. For example, antibodies are purified by removal of contaminating non-immunoglobulin proteins; they are also purified by the removal of immunoglobulin that does not bind to the target molecule. The removal of non-immunoglobulin proteins and/or the removal of immunoglobulins that do not bind to the target molecule results in an increase in the percent of target-reactive immunoglobulins in the sample. In another example, recombinant polypeptides are expressed in bacterial host cells and the polypeptides are purified by the removal of host cell proteins; the percent of recombinant polypeptides is thereby increased in the sample.

### DETAILED DESCRIPTION OF THE INVENTION

The present invention relates to methods for cancer diagnosis therapy as defined in the claims.

### I. Cancer Markers

Experiments conducted during the course of development of the present disclosure identified Angiotensin II receptor type I (AGTR1; Genbank ID AF245699) as being overexpressed in breast cancers. Further experiments indicated that ERBB2 and AGTR I exhibited mutually exclusive overexpression. Additional experiments demonstrated that known AGTR1 antagonists inhibited the proliferation of AGTR1 overexpressing breast cancer cell lines. Yet further experiments identified lipopolysaccharide binding protein (LBP; Genbank ID BC022256) as being overexpressed in breast cancer.

### A. Sample

Any patient sample suspected of containing AGR1 or LBP overexpression may be tested . By way of examples, the sample may be tissue (*e.g.,* a breast biopsy sample or post-surgical tissue), blood, urine, or a fraction thereof (*e.g*., plasma, serum, urine supernatant, urine cell pellet or breast cells). In preferred embodiments, the sample is a tissue sample obtained from a biopsy or following surgery (e.g., lumpectomy or mastectomy).

The patient sample may undergo preliminary processing designed to isolate or enrich the sample for AGTR1 or LBP or cells that contain AGTR1 or LBP. A variety of techniques known to those of ordinary skill in the art may be used for this purpose, including but not limited: centrifugation; immunocapture; cell lysis; and, nucleic acid target capture (*See*, *e.g.,* EP Pat. No. 1 409 727.

### B. DNA and RNA Detection

AGTR1 overexpression is detected as genomic DNA (e.g., copy number amplification) using a variety of nucleic acid techniques known to those of ordinary skill in the art, including but not limited to: nucleic acid sequencing; nucleic acid hybridization; and, nucleic acid amplification.

### 1. Sequencing

Illustrative examples of nucleic acid sequencing techniques include, but are not limited to, chain terminator (Sanger) sequencing and dye terminator sequencing. Those of ordinary skill in the art will recognize that because RNA is less stable in the cell and more prone to nuclease attack experimentally RNA is usually reverse transcribed to DNA before sequencing.

Chain terminator sequencing uses sequence-specific termination of a DNA synthesis reaction using modified nucleotide substrates. Extension is initiated at a specific site on the template DNA by using a short radioactive, or other labeled, oligonucleotide primer complementary to the template at that region. The oligonucleotide primer is extended using a DNA polymerase, standard four deoxynucleotide bases, and a low concentration of one chain terminating nucleotide, most commonly a di-deoxynucleotide. This reaction is repeated in four separate tubes with each of the bases taking turns as the di-deoxynucleotide. Limited incorporation of the chain terminating nucleotide by the DNA polymerase results in a series of related DNA fragments that are terminated only at positions where that particular di-deoxynucleotide is used. For each reaction tube, the fragments are size-separated by electrophoresis in a slab polyacrylamide gel or a capillary tube filled with a viscous polymer. The sequence is determined by reading which lane produces a visualized mark from the labeled primer as you scan from the top of the gel to the bottom.

Dye terminator sequencing alternatively labels the terminators. Complete sequencing can be performed in a single reaction by labeling each of the di-deoxynucleotide chain-terminators with a separate fluorescent dye, which fluoresces at a different wavelength.

### 2. Hybridization

Illustrative examples of nucleic acid hybridization techniques include, but are not limited to, *in situ* hybridization (ISH), microarray, and Southern or Northern blot.

*In situ* hybridization (ISH) is a type of hybridization that uses a labeled complementary DNA or RNA strand as a probe to localize a specific DNA or RNA sequence in a portion or section of tissue (*in situ),* or, if the tissue is small enough, the entire tissue (whole mount ISH). DNA ISH can be used to determine the structure of chromosomes. RNA ISH is used to measure and localize mRNAs and other transcripts within tissue sections or whole mounts. Sample cells and tissues are usually treated to fix the target transcripts in place and to increase access of the probe. The probe hybridizes to the target sequence at elevated temperature, and then the excess probe is washed away. The probe that was labeled with either radio-, fluorescent- or antigen-labeled bases is localized and quantitated in the tissue using either autoradiography, fluorescence microscopy or immunohistochemistry, respectively. ISH can also use two or more probes, labeled with radioactivity or the other non-radioactive labels, to simultaneously detect two or more transcripts.

### 2.1 FISH

AGTR1 or LBP sequences may be detected using fluorescence in *situ* hybridization (FISH). The preferred FISH assays utilize bacterial artificial chromosomes (BACs). These have been used extensively in the human genome sequencing project (see Nature 409: 953-958 (2001)) and clones containing specific BACs are available through distributors that can be located through many sources, *e.g.,* NCBI. Each BAC clone from the human genome has been given a reference name that unambiguously identifies it. These names can be used to find a corresponding GenBank sequence and to order copies of the clone from a distributor.

Specific protocols for performing FISH are well known in the art and can be readily adapted. Guidance regarding methodology may be obtained from many references including: In situ Hybridization: Medical Applications (eds. G. R. Coulton and J. de Belleroche), Kluwer Academic Publishers, Boston (1992); In situ Hybridization: In Neurobiology; Advances in Methodology (eds. J. H. Eberwine, K. L. Valentino, and J. D. Barchas), Oxford University Press Inc., England (1994); In situ Hybridization: A Practical Approach (ed. D. G. Wilkinson), Oxford University Press Inc., England (1992)); Kuo, et al., Am. J. Hum. Genet. 49:112-119 (1991); Klinger, et al., Am. J. Hum. Genet. 51:55-65 (1992); and Ward, et al., Am. J. Hum. Genet. 52:854-865 (1993)). There are also kits that are commercially available and that provide protocols for performing FISH assays (available from *e.g*., Oncor, Inc., Gaithersburg, MD). Patents providing guidance on methodology include U.S. 5,225,326; 5,545,524; 6,121,489 and 6,573,043.

### 2.2 Microarrays

Different kinds of biological assays are called microarrays including, but not limited to: DNA microarrays (e.g., cDNA microarrays and oligonucleotide microarrays); protein microarrays; tissue microarrays; transfection or cell microarrays; chemical compound microarrays; and, antibody microarrays. A DNA microarray, commonly known as gene chip, DNA chip, or biochip, is a collection of microscopic DNA spots attached to a solid surface (e.g., glass, plastic or silicon chip) forming an array for the purpose of expression profiling or monitoring expression levels for thousands of genes simultaneously. The affixed DNA segments are known as probes, thousands of which can be used in a single DNA microarray. Microarrays can be used to identify disease genes by comparing gene expression in disease and normal cells. Microarrays can be fabricated using a variety of technologies, including: printing with fine-pointed pins onto glass slides; photolithography using pre-made masks; photolithography using dynamic micromirror devices; ink-jet printing; or, electrochemistry on microelectrode arrays.

Southern and Northern blotting is used to detect specific DNA or RNA sequences, respectively. DNA or RNA extracted from a sample is fragmented, electrophoretically separated on a matrix gel, and transferred to a membrane filter. The filter bound DNA or RNA is subject to hybridization with a labeled probe complementary to the sequence of interest. Hybridized probe bound to the filter is detected. A variant of the procedure is the reverse Northern blot, in which the substrate nucleic acid that is affixed to the membrane is a collection of isolated DNA fragments and the probe is RNA extracted from a tissue and labeled.

### 3. Amplification

Genomic DNA and mRNA may be amplified prior to or simultaneous with detection. Illustrative non-limiting examples of nucleic acid amplification techniques include, but are not limited to, polymerase chain reaction (PCR), reverse transcription polymerase chain reaction (RT-PCR), transcription-mediated amplification (TMA), ligase chain reaction (LCR), strand displacement amplification (SDA), and nucleic acid sequence based amplification (NASBA). Those of ordinary skill in the art will recognize that certain amplification techniques (e.g., PCR) require that RNA be reversed transcribed to DNA prior to amplification (e.g., RT-PCR), whereas other amplification techniques directly amplify RNA (*e.g.,* TMA and NASBA).

The polymerase chain reaction (U.S. Pat. Nos. 4,683,195, 4,683,202, 4,800,159 and 4,965,188), commonly referred to as PCR, uses multiple cycles of denaturation, annealing of primer pairs to opposite strands, and primer extension to exponentially increase copy numbers of a target nucleic acid sequence. In a variation called RT-PCR, reverse transcriptase (RT) is used to make a complementary DNA (cDNA) from mRNA, and the cDNA is then amplified by PCR to produce multiple copies of DNA. For other various permutations of PCR *see, e.g.,* U.S. Pat. Nos. 4,683,195, 4,683,202 and 4,800,159; Mullis et al., Meth. Enzymol. 155: 335 (1987); and, Murakawa et al., DNA 7: 287 (1988).

Transcription mediated amplification (U.S. Pat. Nos. 5,480,784 and 5,399,491 ), commonly referred to as TMA, synthesizes multiple copies of a target nucleic acid sequence autocatalytically under conditions of substantially constant temperature, ionic strength, and pH in which multiple RNA copies of the target sequence autocatalytically generate additional copies. *See, e.g.,* U.S. Pat. Nos. 5,399,491 and 5,824,518 . In a variation described in U.S. Publ. No. 20060046265 ), TMA optionally incorporates the use of blocking moieties, terminating moieties, and other modifying moieties to improve TMA process sensitivity and accuracy.

The ligase chain reaction (Weiss, R., Science 254: 1292 (1991) ), commonly referred to as LCR, uses two sets of complementary DNA oligonucleotides that hybridize to adjacent regions of the target nucleic acid. The DNA oligonucleotides are covalently linked by a DNA ligase in repeated cycles of thermal denaturation, hybridization and ligation to produce a detectable double-stranded ligated oligonucleotide product.

Strand displacement amplification (Walker, G. et al., Proc. Natl. Acad. Sci. USA 89: 392-396 (1992); U.S. Pat. Nos. 5,270,184 and 5,455,166 ), commonly referred to as SDA, uses cycles of annealing pairs of primer sequences to opposite strands of a target sequence, primer extension in the presence of a dNTPαS to produce a duplex hemiphosphorothioated primer extension product, endonuclease-mediated nicking of a hemimodified restriction endonuclease recognition site, and polymerase-mediated primer extension from the 3' end of the nick to displace an existing strand and produce a strand for the next round of primer annealing, nicking and strand displacement, resulting in geometric amplification of product. Thermophilic SDA (tSDA) uses thermophilic endonucleases and polymerases at higher temperatures in essentially the same method (EP Pat. No. 0 684 315).

Other amplification methods include, for example: nucleic acid sequence based amplification (U.S. Pat. No. 5,130,238), commonly referred to as NASBA; one that uses an RNA replicase to amplify the probe molecule itself (Lizardi et al., BioTechnol. 6: 1197 (1988) ), commonly referred to as Qβ replicase; a transcription based amplification method (Kwoh et al., Proc. Natl. Acad. Sci. USA 86:1173 (1989)); and, self-sustained sequence replication (Guatelli et al., Proc. Natl. Acad. Sci. USA 87: 1874 (1990) ). For further discussion of known amplification methods see Persing, David H., "In Vitro Nucleic Acid Amplification Techniques" in Diagnostic Medical Microbiology: Principles and Applications (Persing et al., Eds.), pp. 51-87 (American Society for Microbiology, Washington, DC (1993)).

### 4. Detection Methods

Non-amplified or amplified AGTR1 or LBP nucleic acids can be detected by any conventional means. For examples, AGTR1 or LBP nucleic acids may be detected by hybridization with a detectably labeled probe and measurement of the resulting hybrids. Illustrative examples of detection methods are described below.

One illustrative detection method, the Hybridization Protection Assay (HPA) involves hybridizing a chemiluminescent oligonucleotide probe (e.g., an acridinium ester-labeled (AE) probe) to the target sequence, selectively hydrolyzing the chemiluminescent label present on unhybridized probe, and measuring the chemiluminescence produced from the remaining probe in a luminometer. *See, e.g.,* U.S. Pat. No. 5,283,174 and Norman C. Nelson et al., Nonisotopic Probing, Blotting, and Sequencing, ch. 17 (Larry J. Kricka ed., 2d ed. 1995).

Another illustrative detection method provides for quantitative evaluation of the amplification process in real-time. Evaluation of an amplification process in "real-time" involves determining the amount of amplicon in the reaction mixture either continuously or periodically during the amplification reaction, and using the determined values to calculate the amount of target sequence initially present in the sample. A variety of methods for determining the amount of initial target sequence present in a sample based on real-time amplification are well known in the art. These include methods disclosed in U.S. Pat. Nos. 6,303,305 and 6,541,205. Another method for determining the quantity of target sequence initially present in a sample, but which is not based on a real-time amplification, is disclosed in U.S. Pat. No. 5,710,029.

Amplification products may be detected in real-time through the use of various self-hybridizing probes, most of which have a stem-loop structure. Such self-hybridizing probes are labeled so that they emit differently detectable signals, depending on whether the probes are in a self-hybridized state or an altered state through hybridization to a target sequence. By way of non-limiting example, "molecular torches" are a type of self-hybridizing probe that includes distinct regions of self-complementarity (referred to as "the target binding domain" and "the target closing domain") which are connected by a joining region (e.g., non-nucleotide linker) and which hybridize to each other under predetermined hybridization assay conditions. Molecular torches may contain single-stranded base regions in the target binding domain that are from 1 to about 20 bases in length and are accessible for hybridization to a target sequence present in an amplification reaction under strand displacement conditions. Under strand displacement conditions, hybridization of the two complementary regions, which may be fully or partially complementary, of the molecular torch is favored, except in the presence of the target sequence, which will bind to the single-stranded region present in the target binding domain and displace all or a portion of the target closing domain. The target binding domain and the target closing domain of a molecular torch include a detectable label or a pair of interacting labels (*e.g.,* luminescent/quencher) positioned so that a different signal is produced when the molecular torch is self-hybridized than when the molecular torch is hybridized to the target sequence, thereby permitting detection of probe:target duplexes in a test sample in the presence of unhybridized molecular torches. Molecular torches and a variety of types of interacting label pairs are *disclosed in U.S. Pat. No. 6,534,274 .

Another example of a detection probe having self-complementarity is a "molecular beacon." Molecular beacons include nucleic acid molecules having a target complementary sequence, an affinity pair (or nucleic acid arms) holding the probe in a closed conformation in the absence of a target sequence present in an amplification reaction, and a label pair that interacts when the probe is in a closed conformation. Hybridization of the target sequence and the target complementary sequence separates the members of the affinity pair, thereby shifting the probe to an open conformation. The shift to the open conformation is detectable due to reduced interaction of the label pair, which may be, for example, a fluorophore and a quencher (*e.g*., DABCYL and EDANS). Molecular beacons are disclosed in U.S. Pat. Nos. 5,925,517 and 6, 150,097.

Other self-hybridizing probes are well known to those of ordinary skill in the art. By way of non-limiting example, probe binding pairs having interacting labels, such as those disclosed in U.S. Pat. No. 5,928,862 might be used. Probe systems used to detect single nucleotide polymorphisms (SNPs) might also be utilized. Additional detection systems include "molecular switches," as disclosed in U.S. Publ. No. 20050042638. Other probes, such as those comprising intercalating dyes and/or fluorochromes, are also useful for detection of amplification products. *See, e.g.,* U.S. Pat. No. 5,814,447 ).

### C. Protein Detection

Disclosed are methods of detecting AGTR1 or LBP protein and levels of AGTR1 or LBP protein. Proteins arc detected using a variety of protein techniques known to those of ordinary skill in the art, including : protein sequencing; and, immunoassays.

### 1. Sequencing

Illustrative examples of protein sequencing techniques include mass spectrometry and Edman degradation.

Mass spectrometry can, in principle, sequence any size protein but becomes computationally more difficult as size increases. A protein is digested by an endoprotease, and the resulting solution is passed through a high pressure liquid chromatography column. At the end of this column, the solution is sprayed out of a narrow nozzle charged to a high positive potential into the mass spectrometer. The charge on the droplets causes them to fragment until only single ions remain. The peptides are then fragmented and the mass-charge ratios of the fragments measured. The mass spectrum is analyzed by computer and often compared against a database of previously sequenced proteins in order to determine the sequences of the fragments. The process is then repeated with a different digestion enzyme, and the overlaps in sequences are used to construct a sequence for the protein.

In the Edman degradation reaction, the peptide to be sequenced is adsorbed onto a solid surface (*e.g.,* a glass fiber coated with polybrene). The Edman reagent, phenylisothiocyanate (PTC), is added to the adsorbed peptide, together with a mildly basic buffer solution of 12% trimethylamine, and reacts with the amine group of the N-terminal amino acid. The terminal amino acid derivative can then be selectively detached by the addition of anhydrous acid. The derivative isomerizes to give a substituted phenylthiohydantoin, which can be washed off and identified by chromatography, and the cycle can be repeated. The efficiency of each step is about 98%, which allows about 50 amino acids to be reliably determined.

### 2. Immunoassays

Illustrative examples of immunoassays include, but are not limited to: immunoprecipitation; Western blot; ELISA; immunohistochemistry; immunocytochemistry; flow cytometry; and, immuno-PCR. Polyclonal or monoclonal antibodies detectably labeled using various techniques known to those of ordinary skill in the art (e.g., colorimetric, fluorescent, chemiluminescent or radioactive) are suitable for use in the immunoassays.

Immunoprecipitation is the technique of precipitating an antigen out of solution using an antibody specific to that antigen. The process can be used to identify protein complexes present in cell extracts by targeting a protein believed to be in the complex. The complexes are brought out of solution by insoluble antibody-binding proteins isolated initially from bacteria, such as Protein A and Protein G. The antibodies can also be coupled to sepharose beads that can easily be isolated out of solution. After washing, the precipitate can be analyzed using mass spectrometry, Western blotting, or any number of other methods for identifying constituents in the complex.

A Western blot, or immunoblot, is a method to detect protein in a given sample of tissue homogenate or extract. It uses gel electrophoresis to separate denatured proteins by mass. The proteins are then transferred out of the gel and onto a membrane, typically polyvinyldiflroride or nitrocellulose, where they are probed using antibodies specific to the protein of interest. As a result, researchers can examine the amount of protein in a given sample and compare levels between several groups.

An ELISA, short for Enzyme-Linked ImmunoSorbent Assay, is a biochemical technique to detect the presence of an antibody or an antigen in a sample. It utilizes a minimum of two antibodies, one of which is specific to the antigen and the other of which is coupled to an enzyme. The second antibody will cause a chromogenic or fluorogenic substrate to produce a signal. Variations of ELISA include sandwich ELISA, competitive ELISA, and ELISPOT. Because the ELISA can be performed to evaluate either the presence of antigen or the presence of antibody in a sample, it is a useful tool both for determining serum antibody concentrations and also for detecting the presence of antigen.

Immunohistochemistry and immunocytochemistry refer to the process of localizing proteins in a tissue section or cell, respectively, via the principle of antigens in tissue or cells binding to their respective antibodies. Visualization is enabled by tagging the antibody with color producing or fluorescent tags. Typical examples of color tags include, but are not limited to, horseradish peroxidase and alkaline phosphatase. Typical examples of fluorophore tags include, but are not limited to, fluorescein isothiocyanate (FITC) or phycoerythrin (PE).

Flow cytometry is a technique for counting, examining and sorting microscopic particles suspended in a stream of fluid. It allows simultaneous multiparametric analysis of the physical and/or chemical characteristics of single cells flowing through an optical/electronic detection apparatus. A beam of light (e.g., a laser) of a single frequency or color is directed onto a hydrodynamically focused stream of fluid. A number of detectors are aimed at the point where the stream passes through the light beam; one in line with the light beam (Forward Scatter or FSC) and several perpendicular to it (Side Scatter (SSC) and one or more fluorescent detectors). Each suspended particle passing through the beam scatters the light in some way, and fluorescent chemicals in the particle may be excited into emitting light at a lower frequency than the light source. The combination of scattered and fluorescent light is picked up by the detectors, and by analyzing fluctuations in brightness at each detector, one for each fluorescent emission peak, it is possible to deduce various facts about the physical and chemical structure of each individual particle. FSC correlates with the cell volume and SSC correlates with the density or inner complexity of the particle (e.g., shape of the nucleus, the amount and type of cytoplasmic granules or the membrane roughness).

Immuno-polymerase chain reaction (IPCR) utilizes nucleic acid amplification techniques to increase signal generation in antibody-based immunoassays. Because no protein equivalence of PCR exists, that is, proteins cannot be replicated in the same manner that nucleic acid is replicated during PCR, the only way to increase detection sensitivity is by signal amplification. The target proteins are bound to antibodies which are directly or indirectly conjugated to oligonucleotides. Unbound antibodies are washed away and the remaining bound antibodies have their oligonucleotides amplified. Protein detection occurs via detection of amplified oligonucleotides using standard nucleic acid detection methods, including real-time methods.

### D. Data Analysis

A computer-based analysis program may be used to translate the raw data generated by the detection assay (e.g., the presence, absence, or amount of AGTR1 or LBP expression) into data of predictive value for a clinician. The clinician can access the predictive data using any suitable means. Thus, the clinician, who is not likely to be trained in genetics or molecular biology, need not understand the raw data. The data is presented directly to the clinician in its most useful form. The clinician is then able to immediately utilize the information in order to optimize the care of the subject.

Contemplated is any method capable of receiving, processing, and transmitting the information to and from laboratories conducting the assays, information provides, medical personal, and subjects. For example, a sample (*e.g.,* a biopsy or a blood or serum sample) is obtained from a subject and submitted to a profiling service (*e.g.,* clinical lab at a medical facility, genomic profiling business, etc.), located in any part of the world (e.g., in a country different than the country where the subject resides or where the information is ultimately used) to generate raw data. Where the sample comprises a tissue or other biological sample, the subject may visit a medical center to have the sample obtained and sent to the profiling center, or subjects may collect the sample themselves (*e.g.*, a urine sample) and directly send it to a profiling center. Where the sample comprises previously determined biological information, the information may be directly sent to the profiling service by the subject (*e.g.,* an information card containing the information may be scanned by a computer and the data transmitted to a computer of the profiling center using an electronic communication systems). Once received by the profiling service, the sample is processed and a profile is produced (*i.e.*, expression data), specific for the diagnostic or prognostic information desired for the subject.

The profile data is then prepared in a format suitable for interpretation by a treating clinician. For example, rather than providing raw expression data, the prepared format may represent a diagnosis or risk assessment (*e.g.*, likelihood of cancer being present) for the subject, along with recommendations for particular treatment options. The data may be displayed to the clinician by any suitable method. For example, the profiling service generates a report that can be printed for the clinician (*e.g.*, at the point of care) or displayed to the clinician on a computer monitor.

The information is first analyzed at the point of care or at a regional facility. The raw data is then sent to a central processing facility for further analysis and/or to convert the raw data to information useful for a clinician or patient. The central processing facility provides the advantage of privacy (all data is stored in a central facility with uniform security protocols), speed, and uniformity of data analysis. The central processing facility can then control the fate of the data following treatment of the subject. For example, using an electronic communication system, the central facility can provide data to the clinician, the subject, or researchers.

The subject may be able to directly access the data using the electronic communication system. The subject may chose further intervention or counseling based on the results. The data may be used for research use. For example, the data may be used to further optimize the inclusion or elimination of markers as useful indicators of a particular condition or stage of disease.

### E. In vivo Imaging

AGTR1 or LBP expression may be detected using *in vivo* imaging techniques, including but not limited to: radionuclide imaging; positron emission tomography (PET); computerized axial tomography, X-ray or magnetic resonance imaging method, fluorescence detection, and chemiluminescent detection. *In vivo* imaging techniques may be used to visualize the presence of or expression of AGTR1 in an animal (e.g., a human or non-human mammal). For example, AGTR1 mRNA or protein may be labeled using a labeled antibody specific for the cancer marker. A specifically bound and labeled antibody can be detected in an individual using an *in vivo* imaging method, including, but not limited to, radionuclide imaging, positron emission tomography, computerized axial tomography, X-ray or magnetic resonance imaging method, fluorescence detection, and chemiluminescent detection. Methods for generating antibodies to the cancer markers are described below.

The *in vivo* imaging methods are useful in the diagnosis of cancers that express AGTR1 or LBP (e.g., breast cancer). *In vivo* imaging is used to visualize the presence of a marker indicative of the cancer. Such techniques allow for diagnosis without the use of an unpleasant biopsy. The *in vivo* imaging methods are also useful for providing prognoses to cancer patients. For example, the presence of a marker indicative of cancers likely to metastasize can be detected. The *in* vivo imaging methods can further be used to detect metastatic cancers in other parts of the body.

Reagents (*e.g.*, antibodies) specific for AGTR1 or LBP may be fluorescently labeled. The labeled antibodies are introduced into a subject (*e.g.*, orally or parenterally). Fluorescently labeled antibodies are detected using any suitable method (*e.g*., using the apparatus described in U.S. Pat. No. 6,198,107

Antibodies may be radioactively labeled. The use of antibodies for *in vivo* diagnosis is well known in the art. Sumerdon et al., (Nucl. Med. Biol 17:247-254 [1990] have described an optimized antibody-chelator for the radioimmunoscintographic imaging of tumors using Indium-111 as the label. Griffin et al., (J Clin Onc 9:631-640 [1991]) have described the use of this agent in detecting tumors in patients suspected of having recurrent colorectal cancer. The use of similar agents with paramagnetic ions as labels for magnetic resonance imaging is known in the art (Lauffer, Magnetic Resonance in Medicine 22:339-342 [1991]). The label used will depend on the imaging modality chosen. Radioactive labels such as Indium-111, Technetium-99m, or Iodine-131 can be used for planar scans or single photon emission computed tomography (SPECT). Positron emitting labels such as Fluorine-19 can also be used for positron emission tomography (PET). For MRI, paramagnetic ions such as Gadolinium (III) or Manganese (II) can be used.

Radioactive metals with half-lives ranging from I hour to 3.5 days are available for conjugation to antibodies, such as scandium-47 (3.5 days) gallium-67 (2.8 days), gallium-68 (68 minutes), technetiium-99m (6 hours), and indium-111 (3.2 days), of which gallium-67, technetium-99m, and indium-111 are preferable for gamma camera imaging, gallium-68 is preferable for positron emission tomography.

A useful method of labeling antibodies with such radiometals is by means of a bifunctional chelating agent, such as diethylenetriaminepentaacetic acid (DTPA), as described, for example, by Khaw et al. (Science 209:295 [1980]) for In-111 and Tc-99m, and by Scheinberg et al. (Science 215:1511 [1982]). Other chelating agents may also be used, but the 1-(p-carboxymethoxybenzyl)EDTA and the carboxycarbonic anhydride of DTPA are advantageous because their use permits conjugation without affecting the antibody's immunoreactivity substantially.

Another method for coupling DPTA to proteins is by use of the cyclic anhydride of DTPA, as described by Hnatowich et al. (Int. J. Appl. Radiat. Isot. 33:327 [1982]) for labeling of albumin with In-111, but which can be adapted for labeling of antibodies. A suitable method of labeling antibodies with Tc-99m which does not use chelation with DPTA is the pretinning method of Crockford et al., (U.S. Pat. No. 4,323,546.

A preferred method of labeling immunoglobulins with Tc-99m is that described by Wong et al. (Int. J. Appl. Radiat. Isot., 29:251 [1978]) for plasma protein, and recently applied successfully by Wong et al. (J. Nucl. Med., 23:229 [1981]) for labeling antibodies.

In the case of the radiometals conjugated to the specific antibody, it is likewise desirable to introduce as high a proportion of the radiolabel as possible into the antibody molecule without destroying its immunospecificity. A further improvement may be achieved by effecting radiolabeling in the presence of the specific cancer marker, to ensure that the antigen binding site on the antibody will be protected. The antigen is separated after labeling.

*in vivo* biophotonic imaging (Xenogen, Almeda, CA) may be utilized for *in vivo* imaging. This real-time *in vivo* imaging utilizes luciferase. The luciferase gene is incorporated into cells, microorganisms, and animals (e.g., as a fusion protein with a cancer marker). When active, it leads to a reaction that emits light. A CCD camera and software is used to capture the image and analyze it.

### F. Compositions & Kits

Compositions for use in the diagnostic methods include, but are not limited to, probes, amplification oligonucleotides, and antibodies. Particularly preferred compositions detect the level of expression of AGTR1 or LBP in a sample.

Any of these compositions, alone or in combination with other compositions , may be provided in the form of a kit. For example, the single labeled probe and pair of amplification oligonucleotides may be provided in a kit for the amplification and detection of AGTR 1 or LBP. Kits may further comprise appropriate controls and/or detection reagents.

The probe and antibody compositions may also be provided in the form of an array.

### II. Therapeutic Methods

Disclosed are methods of customizing cancer (e.g., breast cancer) therapy. For example, the presence or absence of overexpression of AGTR 1 in a sample from a patient is assayed. Patients with overexpression of AGTR1 are then treated with an anti-AGTR1 therapy. The customized treatment methods provide the advantage of therapy directed to a specific target at the molecular level. The use of unnecessary treatments that are not effective (e.g., treating a non AGTR1 1 overexpressing subject with an anti-AGTR1 therapy) can be avoided.

Other therapeutic methods target the overexpression of LBP in breast cancer. For example, known or novel LBP therapeutics find use in the treatment of breast cancers that overexpress LBP.

Therapies are described below. Known AGTR1 antagonists are utilized. The method described herein may be utilized to identify additional therapeutic compositions.

### A. Small Molecule Therapies

Small molecular therapeutics are utilized. The known anti-AGTR1 small molecule drugs are utilized including Losartan (COZAAR), Valsartan (DIOVAN), Eprosartan (TEVETEN), candesartan (ATACAND), irbesartan (AVAPRO), telmisartan (MICARDIS) and Olmesartan (BENICAR). Disclosed are the anti-AGTR1 drugs described in U.S. Pats. 6576652 and 5332820. Additional small molecule therapeutics targeting AGTR1 may be identified, for example, using the drug screening methods of the present disclosure.

### A. RNA Interference and Antisense Therapies

Disclosed is that the expression of AGTR1 or LBP cancer markers is targetted. Composition may comprise oligomeric antisense or RNAi compounds, particularly oligonucleotides (e.g., those identified in the drug screening methods described above), for use in modulating the function of nucleic acid molecules encoding AGTR1 or LBP, ultimately modulating the amount of AGTR1 or LBP expressed.

### 1. RNA Interference (RNAi)

RNAi may be utilized to inhibit AGTR1 or LBP expression. RNAi represents an evolutionary conserved cellular defense for controlling the expression of foreign genes in most eukaryotes, including humans. RNAi is typically triggered by double-stranded RNA (dsRNA) and causes sequence-specific mRNA degradation of single-stranded target RNAs homologous in response to dsRNA. The mediators of mRNA degradation are small interfering RNA duplexes (siRNAs), which are normally produced from long dsRNA by enzymatic cleavage in the cell. siRNAs are generally approximately twenty-one nucleotides in length (e.g. 21-23 nucleotides in length), and have a base-paired structure characterized by two nucleotide 3'-overhangs. Following the introduction of a small RNA, or RNAi, into the cell, it is believed the sequence is delivered to an enzyme complex called RISC (RNA-induced silencing complex). RISC recognizes the target and cleaves it with an endonuclease. It is noted that if larger RNA sequences are delivered to a cell, RNase III enzyme (Dicer) converts longer dsRNA into 21-23 nt ds siRNA fragments. RNAi oligonucleotides may be designed to target AGTR1 or LBP.

Chemically synthesized siRNAs have become powerful reagents for genome-wide analysis of mammalian gene function in cultured somatic cells. Beyond their value for validation of gene function, siRNAs also hold great potential as gene-specific therapeutic agents (Tusch1 and Borkhardt, Molecular Intervent. 2002; 2(3):158-67 ).

The transfection of siRNAs into animal cells results in the potent, long-lasting post-transcriptional silencing of specific genes (Caplen et al, Proc Natl Acad Sci U.S.A. 2001; 98: 9742-7; Elbashir et al., Nature. 2001; 411 :494-8; Elbashir et al., Genes Dev. 2001;15: 188-200; and Elbashir et al., EMBO J. 2001; 20: 6877-88 ). Methods and compositions for performing RNAi with siRNAs are described, for example, in U.S. Pat. 6,506,559.

siRNAs are effective at lowering the amounts of targeted RNA, and by extension proteins, frequently to undetectable levels. The silencing effect can last several months, and is extraordinarily specific, because one nucleotide mismatch between the target RNA and the central region of the siRNA is frequently sufficient to prevent silencing (Brummelkamp et al, Science 2002; 296:550-3; and Holen et al, Nucleic Acids Res. 2002; 30:1757-66.
An important factor in the design of siRNAs is the presence of accessible sites for siRNA binding. Bahoia et al., (J. Biol. Chem., 2003; 278: 15991-15997 ) describe the use of a type of DNA array called a scanning array to find accessible sites in mRNAs for designing effective siRNAs. These arrays comprise oligonucleotides ranging in size from monomers to a certain maximum, usually Comers, synthesized using a physical barrier (mask) by stepwise addition of each base in the sequence. Thus the arrays represent a full oligonucleotide complement of a region of the target gene. Hybridization of the target mRNA to these arrays provides an exhaustive accessibility profile of this region of the target mRNA. Such data are useful in the design of antisense oligonucleotides (ranging from 7mers to 25mers), where it is important to achieve a compromise between oligonucleotide length and binding affinity, to retain efficacy and target specificity (Sohail et al, Nucleic Acids Res., 2001; 29(10): 2041- 2045). Additional methods and concerns for selecting siRNAs are described for example, in WO 05054270, WO05038054A1, WO03070966A2, J Mol Biol. 2005 May 13;348(4):883-93, J Mol Biol. 2005 May 13;348(4):871-81, and Nucleic Acids Res. 2003 Aug 1;31(15):4417-24 . In addition, software (*e.g.*, the MWG online siMAX siRNA design tool) is commercially or publicly available for use in the selection of siRNAs.

### 2. Antisense

AGTR 1 or LBP expression may be modulated using antisense compounds that specifically hybridize with one or more nucleic acids encoding AGTR1 or LBP. The specific hybridization of an oligomeric compound with its target nucleic acid interferes with the normal function of the nucleic acid. This modulation of function of a target nucleic acid by compounds that specifically hybridize to it is generally referred to as "antisense." The functions of DNA to be interfered with include replication and transcription. The functions of RNA to be interfered with include all vital functions such as, for example, translocation of the RNA to the site of protein translation, translation of protein from the RNA, splicing of the RNA to yield one or more mRNA species, and catalytic activity that may be engaged in or facilitated by the RNA. The overall effect of such interference with target nucleic acid function is modulation of the expression of cancer markers "Modulation" means either an increase (stimulation) or a decrease (inhibition) in the expression of a gene. For example, expression may be inhibited to potentially prevent tumor proliferation.

It is preferred to target specific nucleic acids for antisense. "Targeting" an antisense compound to a particular nucleic acid is a multistep process. The process usually begins with the identification of a nucleic acid sequence whose function is to be modulated. This may be, for example, a cellular gene (or mRNA transcribed from the gene) whose expression is associated with a particular disorder or disease state, or a nucleic acid molecule from an infectious agent. The target is a nucleic acid molecule encoding a cancer marker. The targeting process also includes determination of a site or sites within this gene for the antisense interaction to occur such that the desired effect, e.g., detection or modulation of expression of the protein, will result. A preferred intragenic site is the region encompassing the translation initiation or termination codon of the open reading frame (ORF) of the gene. Since the translation initiation codon is typically 5'-AUG (in transcribed mRNA molecules; 5'-ATG in the corresponding DNA molecule), the translation initiation codon is also referred to as the "AUG codon," the "start codon" or the "AUG start codon". A minority of genes have a translation initiation codon having the RNA sequence 5'-GUG, 5'-UUG or 5'-CUG, and 5'-AUA, 5'-ACG and 5'-CUG have been shown to function *in vivo.* Thus, the terms "translation initiation codon" and "start codon" can encompass many codon sequences, even though the initiator amino acid in cach instance is typically methionine (in eukaryotes) or formylmethionine (in prokaryotes). Eukaryotic and prokaryotic genes may have two or more alternative start codons, any one of which may be preferentially utilized for translation initiation in a particular cell type or tissue, or under a particular set of conditions. Start codon" and "translation initiation codon" refer to the codon or codons that are used *in vivo* to initiate translation of an mRNA molecule transcribed from a gene encoding a tumor antigen, regardless of the sequence(s) of such codons.

Translation termination codon (or "stop codon") of a gene may have one of three sequences (*i*.*e.*, 5'-UAA, 5'-UAG and 5'-UGA; the corresponding DNA sequences are 5'-TAA, 5'-TAG and 5'-TGA, respectively). The terms "start codon region" and "translation initiation codon region" refer to a portion of such an mRNA or gene that encompasses from about 25 to about 50 contiguous nucleotides in either direction (*i*.*e.*, 5' or 3') from a translation initiation codon. Similarly, the terms "stop codon region" and "translation termination codon region" refer to a portion of such an mRNA or gene that encompasses from about 25 to about 50 contiguous nucleotides in either direction *(i.e.,* 5' or 3') from a translation termination codon.

The open reading frame (ORF) or "coding region," which refers to the region between the translation initiation codon and the translation termination codon, is also a region that may be targeted effectively. Other target regions include the 5' untranslated region (5' UTR), referring to the portion of an mRNA in the 5' direction from the translation initiation codon, and thus including nucleotides between the 5' cap site and the translation initiation codon of an mRNA or corresponding nucleotides on the gene, and the 3' untranslated region (3' UTR), referring to the portion of an mRNA in the 3' direction from the translation termination codon, and thus including nucleotides between the translation termination codon and 3' end of an mRNA or corresponding nucleotides on the gene. The 5' cap of an mRNA comprises an N7-methylated guanosine residue joined to the 5'-most residue of the mRNA via a 5'-5' triphosphate linkage. The 5' cap region of an mRNA is considered to include the 5' cap structure itself as well as the first 50 nucleotides adjacent to the cap. The cap region may also be a preferred target region.

Although some eukaryotic mRNA transcripts are directly translated, many contain one or more regions, known as "introns," that are excised from a transcript before it is translated. The remaining (and therefore translated) regions are known as "exons" and are spliced together to form a continuous mRNA sequence. mRNA splice sites *(i.e.,* intron-exon junctions) may also be preferred target regions, and are particularly useful *in situ*ations where aberrant splicing is implicated in disease, or where an overproduction of a particular mRNA splice product is implicated in disease. It has also been found that introns can also be effective, and therefore preferred, target regions for antisense compounds targeted, for example, to DNA or pre-mRNA.

Target sites for antisense inhibition are identified using commercially available software programs (*e.g.,* Biognostik, Gottingen, Germany; SysArris Software, Bangalore, India; Antisense Research Group, University of Liverpool, Liverpool, England; GeneTrove, Carlsbad, CA). Target sites for antisense inhibition are identified using the accessible site method described in PCT Publ. No. WO0198537A2.

Once one or more target sites have been identified, oligonucleotides are chosen that are sufficiently complementary to the target (*i.e.*, hybridize sufficiently well and with sufficient specificity) to give the desired effect. For example, antisense oligonucleotides are targeted to or near the start codon.

"Hybridization," with respect to antisense compositions and methods, means hydrogen bonding, which may be Watson-Crick, Hoogsteen or reversed Hoogsteen hydrogen bonding, between complementary nucleoside or nucleotide bases. For example, adenine and thymine are complementary nucleobases that pair through the formation of hydrogen bonds. It is understood that the sequence of an antisense compound need not be 100% complementary to that of its target nucleic acid to be specifically hybridizable. An antisense compound is specifically hybridizable when binding of the compound to the target DNA or RNA molecule interferes with the normal function of the target DNA or RNA to cause a loss of utility, and there is a sufficient degree of complementarity to avoid non-specific binding of the antisense compound to non-target sequences under conditions in which specific binding is desired (*i.e.*, under physiological conditions in the case of *in vivo* assays or therapeutic treatment, and in the case of *in vitro* assays, under conditions in which the assays are performed).

Antisense compounds are commonly used as research reagents and diagnostics. For example, antisense oligonucleotides, which are able to inhibit gene expression with specificity, can be used to elucidate the function of particular genes. Antisense compounds are also used, for example, to distinguish between functions of various members of a biological pathway.

The specificity and sensitivity of antisense is also applied for therapeutic uses. For example, antisense oligonucleotides have been employed as therapeutic moieties in the treatment of disease states in animals and man. Antisense oligonucleotides have been safely and effectively administered to humans and numerous clinical trials are presently underway. It is thus established that oligonucleotides are useful therapeutic modalities that can be configured to be useful in treatment regimes for treatment of cells, tissues, and animals, especially humans.

While antisense oligonucleotides are a preferred form of antisense compound, further disclosed are oligonucleotide mimetics such as are described below. The antisense compounds preferably comprise from about 8 to about 30 nucleobases (*i.e.,* from about 8 to about 30 linked bases), although both longer and shorter sequences may find use. Particularly preferred antisense compounds are antisense oligonucleotides, even more preferably those comprising from about 12 to about 25 nucleobases.

Specific examples of preferred antisense compounds include oligonucleotides containing modified backbones or non-natural internucleoside linkages. As defined in this specification, oligonucleotides having modified backbones include those that retain a phosphorus atom in the backbone and those that do not have a phosphorus atom in the backbone. For the purposes of this specification, modified oligonucleotides that do not have a phosphorus atom in their internucleoside backbone can also be considered to be oligonucleosides.

Preferred modified oligonucleotide backbones include, for example, phosphorothioates, chiral phosphorothioates, phosphorodithioates, phosphotriesters, aminoalkylphosphotriesters, methyl and other alkyl phosphonates including 3'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, and boranophosphates having normal 3'-5' linkages, 2'-5' linked analogs of these, and those having inverted polarity wherein the adjacent pairs of nucleoside units are linked 3'-5' to 5'-3' or 2'-5' to 5'-2'. Various salts, mixed salts and free acid forms are also included.

Preferred modified oligonucleotide backbones that do not include a phosphorus atom therein have backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S and CH₂ component parts.

In other preferred oligonucleotide mimetics, both the sugar and the internucleoside linkage (*i.e.,* the backbone) of the nucleotide units are replaced with novel groups. The base units are maintained for hybridization with an appropriate nucleic acid target compound. One such oligomeric compound, an oligonucleotide mimetic that has been shown to have excellent hybridization properties, is referred to as a peptide nucleic acid (PNA). In PNA compounds, the sugar-backbone of an oligonucleotide is replaced with an amide containing backbone, in particular an aminoethylglycine backbone. The nucleobases are retained and are bound directly or indirectly to aza nitrogen atoms of the amide portion of the backbone. Representative United States patents that teach the preparation of PNA compounds include, but are not limited to, U.S. Pat. Nos.: 5,539,082; 5,714,331; and 5,719,262 . Further teaching of PNA compounds can be found in Nielsen et al., Science 254:1497 (1991).

Most preferred are oligonucleotides with phosphorothioate backbones and oligonucleosides with heteroatom backbones, and in particular --CH₂, --NH--O--CH₂--, --CH₂--N(CH₃)--O--CH₂-- [known as a methylene (methylimino) or MMI backbone], --CH₂--O--N(CH₃)--CH₂--, --CH₂--N(CH₃)--N(CH₃)--CH₂₋₋, and --O--N(CH₃)--CH₂--CH₂-- [wherein the native phosphodiester backbone is represented as --O--P--O--CH₂--] of the above referenced U.S. Pat. No. 5,489,677, and the amide backbones of the above referenced U.S. Pat. No. 5,602,240. Also preferred are oligonucleotides having morpholino backbone structures of the above-referenced U.S. Pat. No. 5,034,506.

Modified oligonucleotides may also contain one or more substituted sugar moieties. Preferred oligonucleotides comprise one of the following at the 2' position: OH; F; O-, S-, or N-alkyl; O-, S-, or N-alkenyl; O-, S- or N-alkynyl; or O-alkyl-O-alkyl, wherein the alkyl, alkenyl and alkynyl may be substituted or unsubstituted C₁ to C₁₀ alkyl or C₂ to C₁₀ alkenyl and alkynyl. Particularly preferred are O[(CH₂)ₙO]ₘCH₃, O(CH₂)ₙOCH₃, O(CH₂)ₙNH₂, O(CH₂)ₙCH₃, O(CH₂)ₙONH₂, and O(CH₂)ₙON[(CH₂)ₙCH₃)]₂, where n and m arc from 1 to about 10. Other preferred oligonucleotides comprise one of the following at the 2' position: C₁ to C₁₀ lower alkyl, substituted lowcr alkyl, alkaryl, aralkyl, O-alkaryl or O-aralkyl, SH, SCH₃, OCN, Cl, Br, CN, CF₃, OCF₃, SOCH₃, SO₂CH₃, ONO₂, NO₂, N3, NH2, heterocycloalkyl, heterocycloalkaryl, aminoalkylamino, polyalkylamino, substituted silyl, an RNA cleaving group, a reporter group, an intercalator, a group for improving the pharmacokinetic properties of an oligonucleotide, or a group for improving the pharmacodynamic properties of an oligonucleotide, and other substituents having similar properties. A preferred modification includes 2'-methoxyethoxy (2'-O--CH₂CH₂OCH₃, also known as 2'-O-(2-methoxyethyl) or 2'-MOE) (Martin et al., Helv. Chim. Acta 78:486 [1995]) *i.e.,* an alkoxyalkoxy group. A further preferred modification includes 2'-dimethylaminooxyethoxy (*i*.*e.*, a O(CH₂)₂ON(CH₃)₂ group), also known as 2'-DMAOE, and 2'-dimethylaminoethoxyethoxy (also known in the art as 2'-O-dimethylaminoethoxyethyl or 2'-DMAEOE), *i.e*., 2'-O--CH₂--O--CH₂--N(CH₂)₂.

Other preferred modifications include 2'-methoxy(2'-O--CH₃), 2'-aminopropoxy(2'-OCH₂CH₂CH₂NH₂) and 2'-fluoro (2'-F). Similar modifications may also be made at other positions on the oligonucleotide, particularly the 3' position of the sugar on the 3' terminal nucleotide or in 2'-5' linked oligonucleotides and the 5' position of 5' terminal nucleotide. Oligonucleotides may also have sugar mimetics such as cyclobutyl moieties in place of the pentofuranosyl sugar.

Oligonucleotides may also include nucleobase (often referred to in the art simply as "base") modifications or substitutions. As used herein, "unmodified" or "natural" nucleobases include the purine bases adenine (A) and guanine (G), and the pyrimidine bases thymine (T), cytosine (C) and uracil (U). Modified nucleobases include other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 8-azaguanine and 8-azaadenine, 7-deazaguanine and 7-deazaadenine and 3-deazaguanine and 3-deazaadenine. Further nucleobases include those disclosed in U.S. Pat. No. 3,687,808. Certain of these nucleobases are particularly useful for increasing the binding affinity of the oligomeric compounds. These include 5-substituted pyrimidines, 6-azapyrimidines and N-2, N-6 and O-6 substituted purines, including 2-aminopropyladenine, 5-propynyluracil and 5-propynylcytosine. 5-mcthylcytosine substitutions have been shown to increase nucleic acid duplex stability by 0.6-1.2. °C and are presently preferred base substitutions, even more particularly when combined with 2'-O-methoxyethyl sugar modifications.

Another modification of the oligonucleotides involves chemically linking to the oligonucleotide one or more moieties or conjugates that enhance the activity, cellular distribution or cellular uptake of the oligonucleotide. Such moieties include but are not limited to lipid moieties such as a cholesterol moiety, cholic acid, a thioether, (*e.g*., hexyl-S-tritylthiol), a thiocholesterol, an aliphatic chain, (*e.g.*, dodecandiol or undecyl residues), a phospholipid, (*e.g*., di-hexadecyl-rac-glycerol or triethylammonium 1,2-di-O-hexadecyl-rac-glycero-3-H-phosphonate), a polyamine or a polyethylene glycol chain or adamantane acetic acid, a palmityl moiety, or an octadecylamine or hexylamino-carbonyl-oxycholesterol moiety.

One skilled in the relevant art knows well how to generate oligonucleotides containing the above-described modifications. Any suitable modification or substitution may be utilized.

It is not necessary for all positions in a given compound to be uniformly modified, and in fact more than one of the aforementioned modifications may be incorporated in a single compound or even at a single nucleoside within an oligonucleotide. Disclosed are antisense compounds that are chimeric compounds. "Chimeric" antisense compounds or "chimeras," are antisense compounds, particularly oligonucleotides, which contain two or more chemically distinct regions, each made up of at least one monomer unit, *i.e.,* a nucleotide in the case of an oligonucleotide compound. These oligonucleotides typically contain at least one region wherein the oligonucleotide is modified so as to confer upon the oligonucleotide increased resistance to nuclease degradation, increased cellular uptake, and/or increased binding affinity for the target nucleic acid. An additional region of the oligonucleotide may serve as a substrate for enzymes capable of cleaving RNA:DNA or RNA:RNA hybrids. By way of example, RNaseH is a cellular endonuclease that cleaves the RNA strand of an RNA:DNA duplex. Activation of RNase H, therefore, results in cleavage of the RNA target, thereby greatly enhancing the efficiency of oligonucleotide inhibition of gene expression. Consequently, comparable results can often be obtained with shorter oligonucleotides when chimeric oligonucleotides are used, compared to phosphorothioate deoxyoligonucleotides hybridizing to the same target region. Cleavage of the RNA target can be routinely detected by gel electrophoresis and, if necessary, associated nucleic acid hybridization techniques known in the art.

Chimeric antisense compounds may be formed as composite structures of two or more oligonucleotides, modified oligonucleotides, oligonucleosides and/or oligonucleotide mimetics as described above.

Disclosed are pharmaceutical compositions formulations that include the antisense compounds as described below.

### B. Gene Therapy

Disclosed is the use of any genetic manipulation for use in modulating the expression of AGTR1 or LBP cancer markers. Examples of genetic manipulation include, but are not limited to, gene knockout (*e.g*., removing the AGTR1 or LBP gene from the chromosome using, for example, recombination), expression of antisense constructs with or without inducible promoters, and the like. Delivery of nucleic acid construct to cells *in vitro* or *in vivo* may be conducted using any suitable method. A suitable method is one that introduces the nucleic acid construct into the cell such that the desired event occurs (*e.g*., expression of an antisense construct). Genetic therapy may also be used to deliver siRNA or other interfering molecules that are expressed *in vivo* (*e.g.,* upon stimulation by an inducible promoter (*e.g.*, an androgen-responsive promoter)).

Introduction of molecules carrying genetic information into cells is achieved by any of various methods including, but not limited to, directed injection of naked DNA constructs, bombardment with gold particles loaded with said constructs, and macromolecule mediated gene transfer using, for example, liposomes, biopolymers, and the like. Preferred methods use gene delivery vehicles derived from viruses, including, but not limited to, adenoviruses, retroviruses, vaccinia viruses, and adeno-associated viruses. Because of the higher efficiency as compared to retroviruses, vectors derived from adenoviruses are the preferred gene delivery vehicles for transferring nucleic acid molecules into host cells *in vivo.* Adenoviral vectors have been shown to provide very efficient *in vivo* gene transfer into a variety of solid tumors in animal models and into human solid tumor xenografts in immune-deficient mice. Examples of adenoviral vectors and methods for gene transfer are described in PCT publications WO 00/12738 and WO 00/09675 and U.S. Pat. Appl. Nos. 6,033,908, 6,019,978, 6,001,557, 5,994,132, 5,994,128, 5,994,106, 5,981,225, 5,885,808, 5,872,154, 5,830,730, and 5,824,544. .

Vectors may be administered to subject in a variety of ways. For example , vectors are administered into tumors or tissue associated with tumors using direct injection. Administration is via the blood or lymphatic circulation *(See e.g.,* PCT publication 99/02685 ). Exemplary dose levels of adenoviral vector are preferably 10⁸ to 10¹¹ vector particles added to the perfusate.

### C. Antibody Therapy

Disclosed are antibodies that target breast tumors that overexpress AGTR1 or LBP. Any suitable antibody (*e.g.*, monoclonal, polyclonal, or synthetic) may be utilized in the therapeutic methods disclosed herein. The antibodies used for cancer therapy may be humanized antibodies. Methods for humanizing antibodies are well known in the art *(See e.g.,* U.S. Pat. Nos. 6,180,370, 5,585,089, 6,054,297, and 5,565,332 ).

The therapeutic antibodies may comprise an antibody generated against AGTR1 or LBP, wherein the antibody is conjugated to a cytotoxic agent. A tumor specific therapeutic agent may be generated that does not target normal cells, thus reducing many of the detrimental side effects of traditional chemotherapy. For certain applications, it is envisioned that the therapeutic agents will be pharmacologic agents that will serve as useful agents for attachment to antibodies, particularly cytotoxic or otherwise anticellular agents having the ability to kill or suppress the growth or cell division of the of of endothelial cells. Disclosed is the use of any pharmacologic agent that can be conjugated to an antibody, and delivered in active form. Exemplary anticellular agents include chemotherapeutic agents, radioisotopes, and cytotoxins. The therapeutic antibodies may include a variety of cytotoxic moieties, including but not limited to, radioactive isotopes (*e.g*., iodine-131, iodine-123, technicium-99m, indium-111, rhenium-188, rhenium-186, gallium-67, copper-67, yttrium-90, iodine-125 or astatine-211), hormones such as a steroid, antimetabolites such as cytosines (*e.g*., arabinoside, fluorouracil, methotrexate or aminopterin; an anthracycline; mitomycin C), vinca alkaloids (*e.g.*, demecolcine; etoposide, mithramycin), and antitumor alkylating agent such as chlorambucil or melphalan. Further disclosed are agents such as a coagulant, a cytokinc, growth factor, bacterial endotoxin or the lipid A moiety of bacterial endotoxin. For example, therapeutic agents will include plant-, fungus- or bacteria-derived toxin, such as an A chain toxins, a ribosome inactivating protein, α-sarcin, aspergillin, restrictocin, a ribonuclease, diphtheria toxin or pseudomonas exotoxin, to mention just a few examples. Preferred, deglycosylated ricin A chain is utilized.

In any event, it is proposed that agents such as these may, if desired, be successfully conjugated to an antibody, in a manner that will allow their targeting, internalization, release or presentation to blood components at the site of the targeted tumor cells as required using known conjugation technology *(See, e.g.,* Ghose et al., Methods Enzymol., 93:280 [1983]).

For example, disclosed are immunotoxins targeted a cancer marker (*e.g.*, AGTR1). Immunotoxins are conjugates of a specific targeting agent typically a tumor-directed antibody or fragment, with a cytotoxic agent, such as a toxin moiety. The targeting agent directs the toxin to, and thereby selectively kills, cells carrying the targeted antigen. Therapeutic antibodies may employ crosslinkers that provide high *in vivo* stability (Thorpe et al., Cancer Res., 48:6396 [1988]).

By treatment of solid tumors, antibodies are designed to have a cytotoxic or otherwise anticellular effect against the tumor vasculature, by suppressing the growth or cell division of the vascular endothelial cells. This attack is intended to lead to a tumor-localized vascular collapse, depriving the tumor cells, particularly those tumor cells distal of the vasculature, of oxygen and nutrients, ultimately leading to cell death and tumor necrosis.

Antibody based therapeutics may be formulated as pharmaceutical compositions as described below. Administration of an antibody composition results in a measurable decrease in cancer (*e.g.*, decrease or elimination of tumor).

### D. Pharmaceutical Compositions

Disclosed are pharmaceutical compositions (*e.g.*, comprising pharmaceutical agents that modulate the expression or activity of AGTR1 or LBP). The pharmaceutical compositions may be administered in a number of ways depending upon whether local or systemic treatment is desired and upon the area to be treated. Administration may be topical (including ophthalmic and to mucous membranes including vaginal and rectal delivery), pulmonary (*e.g*., by inhalation or insufflation of powders or aerosols, including by nebulizer; intratracheal, intranasal, epidermal and transdermal), oral or parenteral. Parenteral administration includes intravenous, intraarterial, subcutaneous, intraperitoneal or intramuscular injection or infusion; or intracranial, *e.g.,* intrathecal or intraventricular, administration.

Pharmaceutical compositions and formulations for topical administration may include transdermal patches, ointments, lotions, creams, gels, drops, suppositories, sprays, liquids and powders. Conventional pharmaceutical carriers, aqueous, powder or oily bases, thickeners and the like may be necessary or desirable.

Compositions and formulations for oral administration include powders or granules, suspensions or solutions in water or non-aqueous media, capsules, sachets or tablets. Thickeners, flavoring agents, diluents, emulsifiers, dispersing aids or binders may be desirable.

Compositions and formulations for parenteral, intrathecal or intraventricular administration may include sterile aqueous solutions that may also contain buffers, diluents and other suitable additives such as, but not limited to, penetration enhancers, carrier compounds and other pharmaceutically acceptable carriers or excipients.

Pharmaceutical compositions include, but are not limited to, solutions, emulsions, and liposome-containing formulations. These compositions may be generated from a variety of components that include, but are not limited to, preformed liquids, self-emulsifying solids and self-emulsifying semisolids.

The pharmaceutical formulations , which may conveniently be presented in unit dosage form, may be prepared according to conventional techniques well known in the pharmaceutical industry. Such techniques include the step of bringing into association the active ingredients with the pharmaceutical carrier(s) or excipient(s). In general the formulations are prepared by uniformly and intimately bringing into association the active ingredients with liquid carriers or finely divided solid carriers or both, and then, if necessary, shaping the product.

The compositions may be formulated into any of many possible dosage forms such as, but not limited to, tablets, capsules, liquid syrups, soft gels, suppositories, and enemas. The compositions may also be formulated as suspensions in aqueous, non-aqueous or mixed media. Aqueous suspensions may further contain substances that increase the viscosity of the suspension including, for example, sodium carboxymethylcellulose, sorbitol and/or dextran. The suspension may also contain stabilizers.

The pharmaceutical compositions may be formulated and used as foams. Pharmaceutical foams include formulations such as, but not limited to, emulsions, microemulsions, creams, jellies and liposomes. While basically similar in nature these formulations vary in the components and the consistency of the final product.

Agents that enhance uptake of oligonucleotides at the cellular level may also be added to the pharmaceutical and other compositions. For example, cationic lipids, such as lipofectin (U.S. Pat. No. 5,705,188), cationic glycerol derivatives, and polycationic molecules, such as polylysine (WO 97/30731), also enhance the cellular uptake of oligonucleotides.

The compositions may additionally contain other adjunct components conventionally found in pharmaceutical compositions. Thus, for example, the compositions may contain additional, compatible, pharmaceutically-active materials such as, for example, antipruritics, astringents, local anesthetics or anti-inflammatory agents, or may contain additional materials useful in physically formulating various dosage forms of the compositions, such as dyes, flavoring agents, preservatives, antioxidants, opacifiers, thickening agents and stabilizers. However, such materials, when added, should not unduly interfere with the biological activities of the components of the compositions. The formulations can be sterilized and, if desired, mixed with auxiliary agents, *e.g.,* lubricants, preservatives, stabilizers, wetting agents, emulsifiers, salts for influencing osmotic pressure, buffers, colorings, flavorings and/or aromatic substances and the like which do not deleteriously interact with the nucleic acid(s) of the formulation.

Certain embodiments provide pharmaceutical compositions containing (a) one or more antisense compounds and (b) one or more other chemotherapeutic agents that function by a non-antisense mechanism. Examples of such chemotherapeutic agents include, but are not limited to, anticancer drugs such as daunorubicin, dactinomycin, doxorubicin, bleomycin, mitomycin, nitrogen mustard, chlorambucil, melphalan, cyclophosphamide, 6-mercaptopurine, 6-thioguanine, cytarabine (CA), 5-fluorouracil (5-FU), floxuridine (5-FUdR), methotrexate (MTX), colchicine, vincristine, vinblastine, etoposide, teniposide, cisplatin and diethylstilbestrol (DES). Anti-inflammatory drugs, including but not limited to nonsteroidal anti-inflammatory drugs and corticosteroids, and antiviral drugs, including but not limited to ribivirin, vidarabine, acyclovir and ganciclovir, may also be combined in compositions. Other non-antisense chemotherapeutic agents may also be used. Two or more combined compounds may be used together or sequentially.

Dosing is dependent on severity and responsiveness of the disease state to be treated, with the course of treatment lasting from several days to several months, or until a cure is effected or a diminution of the disease state is achieved. Optimal dosing schedules can be calculated from measurements of drug accumulation in the body of the patient. The administering physician can easily determine optimum dosages, dosing methodologies and repetition rates. Optimum dosages may vary depending on the relative potency of individual oligonucleotides, and can generally be estimated based on EC₅₀s found to be effective in *in vitro* and *in vivo* animal models or based on the examples described herein. In general, dosage is from 0.01 µg to 100 g per kg of body weight, and may be given once or more daily, weekly, monthly or yearly. The treating physician can estimate repetition rates for dosing based on measured residence times and concentrations of the drug in bodily fluids or tissues. Following successful treatment, it may be desirable to have the subject undergo maintenance therapy to prevent the recurrence of the disease state, wherein the oligonucleotide is administered in maintenance doses, ranging from 0.01 µg to 100 g per kg of body weight, once or more daily, to once every 20 years.

### III. Antibodies

AGTR1 or LBP protein, including fragments, derivatives and analogs thereof, may be used as immunogens to produce antibodies having use in the diagnostic, research, and therapeutic methods described below. The antibodies may be polyclonal or monoclonal, chimeric, humanized, single chain or Fab fragments. Various procedures known to those of ordinary skill in the art may be used for the production and labeling of such antibodies and fragments. *See, e.g.,* Bums, ed., Immunochemical Protocols, 3rd ed., Humana Press (2005); Harlow and Lane, Antibodies: A Laboratory Manual, Cold Spring Harbor Laboratory (1988); Kozbor et al., Immunology Today 4: 72 (1983); Köhler and Milstein, Nature 256: 495(1975).

### V. Drug Screening Applications

Disclosed are drug screening assays (*e.g.*, to screen for anticancer drugs). The screening methods utilize cancer markers (*e.g.,* AGTR1 or LBP). Disclosed are methods of screening for compounds that alter (*e.g.*, decrease) the expression of cancer marker genes. The compounds or agents may interfere with transcription, by interacting, for example, with the promoter region. The compounds or agents may interfere with mRNA produced from AGTR1 or LBP (*e.g*., by RNA interference, antisense technologies, etc.). The compounds or agents may interfere with pathways that are upstream or downstream of the biological activity of AGTR1 or LBP. Candidate compounds are antisense or interfering RNA agents (*e.g*., oligonucleotides) directed against cancer markers or candidate compounds are antibodies or small molecules that specifically bind to a cancer marker regulator or expression products and inhibit its biological function.

In one screening method, candidate compounds are evaluated for their ability to alter cancer marker expression by contacting a compound with a cell expressing a cancer marker and then assaying for the effect of the candidate compounds on expression. The effect of candidate compounds on expression of a cancer marker gene may be assayed for by detecting the level of cancer marker mRNA expressed by the cell. mRNA expression can be detected by any suitable method. The effect of candidate compounds on expression of cancer marker genes may be assayed by measuring the level of polypeptide encoded by the cancer markers. The level of polypeptide expressed can be measured using any suitable method, including but not limited to, those disclosed herein.

Specifically, disclosed are screening methods for identifying modulators, *i.e.,* candidate or test compounds or agents (*e.g*., proteins, peptides, peptidomimetics, peptoids, small molecules or other drugs) which bind to cancer markers , have an inhibitory (or stimulatory) effect on, for example, cancer marker expression or cancer marker activity, or have a stimulatory or inhibitory effect on, for example, the expression or activity of a cancer marker substrate. Compounds thus identified can be used to modulate the activity of target gene products (*e.g.*, cancer marker genes) either directly or indirectly in a therapeutic protocol, to elaborate the biological function of the target gene product, or to identify compounds that disrupt normal target gene interactions. Compounds that inhibit the activity or expression of cancer markers are useful in the treatment of proliferative disorders, *e.g.,* cancer, particularly prostate cancer.

Further disclosed are assays for screening candidate or test compounds that are substrates of a cancer marker protein or polypeptide or a biologically active portion thereof. Also disclosed are assays for screening candidate or test compounds that bind to or modulate the activity of a cancer marker protein or polypeptide or a biologically active portion thereof.

The test compounds can be obtained using any of the numerous approaches in combinatorial library methods known in the art, including biological libraries; peptoid libraries (libraries of molecules having the functionalities of peptides, but with a novel, non-peptide backbone, which are resistant to enzymatic degradation but which nevertheless remain bioactive; see, *e.g.,* Zuckennann et al., J. Med. Chem. 37: 2678-85 [1994]); spatially addressable parallel solid phase or solution phase libraries; synthetic library methods requiring deconvolution; the'one-bead one-compound' library method; and synthetic library methods using affinity chromatography selection. The biological library and peptoid library approaches are preferred for use with peptide libraries, while the other four approaches are applicable to peptide, non-peptide oligomer or small molecule libraries of compounds (Lam (1997) Anticancer Drug Des. 12:145).

Examples of methods for the synthesis of molecular libraries can be found in the art, for example in: DeWitt et al., Proc. Natl. Acad. Sci. U.S.A. 90:6909 [1993]; Erb et al., Proc. Nad. Acad. Sci. USA 91:11422 [1994]; Zuckermann et al., J. Med. Chem. 37:2678 [1994]; Cho et al., Science 261:1303 [1993]; Carrell et al., Angew. Chem. Int. Ed. Engl. 33.2059 [1994]; Carell et al., Angew. Chem. Int. Ed. Engl. 33:2061 [1994]; and Gallop et al., J. Med. Chem. 37:1233 [1994].

Libraries of compounds may be presented in solution (*e.g.*, Houghten, Biotechniques 13:412-421 [1992]), or on beads (Lam, Nature 354:82-84 [1991]), chips (Fodor, Nature 364:555-556 [1993]), bacteria or spores (U.S. Pat. No. 5,223,409 ), plasmids (Cull et al., Proc. Nad. Acad. Sci. USA 89:18651869 [1992]) or on phage (Scott and Smith, Science 249:386-390 [1990]; Devlin Science 249:404-406 [1990]; Cwirla et al., Proc. NatI. Acad. Sci. 87:6378-6382 [1990]; Felici, J. Mol. Biol. 222:301 [1991]).

An assay is a cell-based assay in which a cell that expresses a cancer marker mRNA or protein or biologically active portion thereof is contacted with a test compound, and the ability of the test compound to the modulate cancer marker's activity is determined. Determining the ability of the test compound to modulate cancer marker activity can be accomplished by monitoring, for example, changes in enzymatic activity, destruction or mRNA, or the like.

The ability of the test compound to modulate cancer marker binding to a compound, *e.g.,* a cancer marker substrate or modulator, can also be evaluated. This can be accomplished, for example, by coupling the compound, *e.g*., the substrate, with a radioisotope or enzymatic label such that binding of the compound, *e.g.,* the substrate, to a cancer marker can be determined by detecting the labeled compound, *e.g.,* substrate, in a complex.

Alternatively, the cancer marker is coupled with a radioisotope or enzymatic label to monitor the ability of a test compound to modulate cancer marker binding to a cancer marker substrate in a complex. For example, compounds (*e.g.*, substrates) can be labeled with ¹²⁵I, ³⁵S ¹⁴C or ³H, either directly or indirectly, and the radioisotope detected by direct counting of radioemmission or by scintillation counting. Alternatively, compounds can be enzymatically labeled with, for example, horseradish peroxidase, alkaline phosphatase, or luciferase, and the enzymatic label detected by determination of conversion of an appropriate substrate to product.

The ability of a compound (*e.g.*, a cancer marker substrate) to interact with a cancer marker with or without the labeling of any of the interactants can be evaluated. For example, a microphysiorneter can be used to detect the interaction of a compound with a cancer marker without the labeling of either the compound or the cancer marker (McConnell et al. Science 257:1906-1912 [1992]). As used herein, a "microphysiometer" (*e.g.*, Cytosensor) is an analytical instrument that measures the rate at which a cell acidifies its environment using a light-addressable potentiometric sensor (LAPS). Changes in this acidification rate can be used as an indicator of the interaction between a compound and cancer markers.

Further disclosed is assay in which a cancer marker a a cancer marker protein or biologically active portion thereof is contacted with a test compound and the ability of the test compound to bind to the cancer marker protein, mRNA, or biologically active portion thereof is evaluated. Preferred biologically active portions of the cancer marker proteins or mRNA to be used in assays include fragments that participate in interactions with substrates or other proteins, *e.g.,* fragments with high surface probability scores.

Cell-free assays involve preparing a reaction mixture of the target gene protein and the test compound under conditions and for a time sufficient to allow the two components to interact and bind, thus forming a complex that can be removed and/or detected.

The interaction between two molecules can also be detected, *e.g.,* using fluorescence energy transfer (FRET) (see, for example, Lakowicz et al., U.S. Pat. No. 5,631,169; Stavrianopoulos et al., U.S. Pat. No. 4,968,103 ). A fluorophore label is selected such that a first donor molecule's emitted fluorescent energy will be absorbed by a fluorescent label on a second, 'acceptor' molecule, which in turn is able to fluoresce due to the absorbed energy.

Alternately, the 'donor' protein molecule may simply utilize the natural fluorescent energy of tryptophan residues. Labels are chosen that emit different wavelengths of light, such that the 'acceptor' molecule label may be differentiated from that of the 'donor'. Since the efficiency of energy transfer between the labels is related to the distance separating the molecules, the spatial relationship between the molecules can be assessed. In a situation in which binding occurs between the molecules, the fluorescent emission of the'acceptor' molecule label should be maximal. A FRET binding event can be conveniently measured through standard fluorometric detection means well known in the art (*e.g.*, using a fluorimeter).

Determining the ability of the cancer marker protein or mRNA to bind to a target molecule can be accomplished using real-time Biomolecular Interaction Analysis (BIA) (*see, e.g.,* Sjolander and Urbaniczky, Anal. Chem. 63:2338-2345 [1991] and Szabo et al. Curr. Opin. Struct. Biol. 5:699-705 [1995]). "Surface plasmon resonance" or "BIA" detects biospecific interactions in real time, without labeling any of the interactants (*e.g*., BlAcore). Changes in the mass at the binding surface (indicative of a binding event) result in alterations of the refractive index of light near the surface (the optical phenomenon of surface plasmon resonance (SPR)), resulting in a detectable signal that can be used as an indication of real-time reactions between biological molecules.

The target gene product or the test substance may be anchored onto a solid phase. The target gene product/test compound complexes anchored on the solid phase can be detected at the end of the reaction. Preferably, the target gene product can be anchored onto a solid surface, and the test compound, (which is not anchored), can be labeled, either directly or indirectly, with detectable labels discussed herein.

It may be desirable to immobilize cancer markers, an anti-cancer marker antibody or its target molecule to facilitate separation of complexed from non-complexed forms of one or both of the proteins, as well as to accommodate automation of the assay. Binding of a test compound to a cancer marker protein, or interaction of a cancer marker protein with a target molecule in the presence and absence of a candidate compound, can be accomplished in any vessel suitable for containing the reactants. Examples of such vessels include microtiter plates, test tubes, and micro-centrifuge tubes. A fusion protein can be provided which adds a domain that allows one or both of the proteins to be bound to a matrix. For example, glutathione-S-transferase-cancer marker fusion proteins or glutathione-S-transferase/target fusion proteins can be adsorbed onto glutathione Sepharose beads (Sigma Chemical, St. Louis, MO) or glutathione-derivatized microtiter plates, which are then combined with the test compound or the test compound and either the non-adsorbed target protein or cancer marker protein, and the mixture incubated under conditions conducive for complex formation (*e.g.*, at physiological conditions for salt and pH). Following incubation, the beads or microtiter plate wells are washed to remove any unbound components, the matrix immobilized in the case of beads, complex determined either directly or indirectly, for example, as described above.

Alternatively, the complexes can be dissociated from the matrix, and the level of cancer markers binding or activity determined using standard techniques. Other techniques for immobilizing either cancer markers protein or a target molecule on matrices include using conjugation of biotin and streptavidin. Biotinylated cancer marker protein or target molecules can be prepared from biotin-NHS (N-hydroxy-succinimide) using techniques known in the art (*e.g.*, biotinylation kit, Pierce Chemicals, Rockford, EL), and immobilized in the wells of streptavidin-coated 96 well plates (Pierce Chemical).

In order to conduct the assay, the non-immobilized component is added to the coated surface containing the anchored component. After the reaction is complete, unreacted components are removed (*e.g*., by washing) under conditions such that any complexes formed will remain immobilized on the solid surface. The detection of complexes anchored on the solid surface can be accomplished in a number of ways. Where the previously non-immobilized component is pre-labeled, the detection of label immobilized on the surface indicates that complexes were formed. Where the previously non-immobilized component is not pre-labeled, an indirect label can be used to detect complexes anchored on the surface; *e.g*., using a labeled antibody specific for the immobilized component (the antibody, in turn, can be directly labeled or indirectly labeled with, *e.g.*, a labeled anti-IgG antibody).

This assay is performed utilizing antibodies reactive with cancer marker protein or target molecules but which do not interfere with binding of the cancer markers protein to its target molecule. Such antibodies can be derivatized to the wells of the plate, and unbound target or cancer markers protein trapped in the wells by antibody conjugation. Methods for detecting such complexes, in addition to those described above for the GST-immobilized complexes, include immunodetection of complexes using antibodies reactive with the cancer marker protein or target molecule, as well as enzyme-linked assays which rely on detecting an enzymatic activity associated with the cancer marker protein or target molecule.

Alternatively, cell free assays can be conducted in a liquid phase. In such an assay, the reaction products are separated from unreacted components, by any of a number of standard techniques, including, but not limited to: differential centrifugation (see, for example, Rivas and Minton, Trends Biochem Sci 18:284-7 [1993]); chromatography (gel filtration chromatography, ion-exchange chromatography); electrophoresis (*see, e.g.,* Ausubel et al., eds. Current Protocols in Molecular Biology 1999, J. Wiley: New York.); and immunoprecipitation (*see,* for example, Ausubel et al., eds. Current Protocols in Molecular Biology 1999, J. Wiley: New York). Such resins and chromatographic techniques are known to one skilled in the art *(See e.g.,* Heegaard J. Mol. Recognit 11:141-8 [1998]; Hageand Tweed J. Chromatogr. Biomed. Sci. App1 699:499-525 [1997]). Further, fluorescence energy transfer may also be conveniently utilized, as described herein, to detect binding without further purification of the complex from solution.

The assay can include contacting the cancer markers protein, mRNA, or biologically active portion thereof with a known compound that binds the cancer marker to form an assay mixture, contacting the assay mixture with a test compound, and determining the ability of the test compound to interact with a cancer marker protein or mRNA, wherein determining the ability of the test compound to interact with a cancer marker protein or mRNA includes determining the ability of the test compound to preferentially bind to cancer markers or biologically active portion thereof, or to modulate the activity of a target molecule, as compared to the known compound.

To the extent that cancer markers can, *in vivo,* interact with one or more cellular or extracellular macromolecules, such as proteins, inhibitors of such an interaction are useful. A homogeneous assay can be used can be used to identify inhibitors.

For example, a preformed complex of the target gene product and the interactive cellular or extracellular binding partner product is prepared such that either the target gene products or their binding partners are labeled, but the signal generated by the label is quenched due to complex formation (see, *e.g.,* U.S. Pat. No. 4,109,496 , which utilizes this approach for immunoassays). The addition of a test substance that competes with and displaces one of the species from the preformed complex will result in the generation of a signal above background. In this way, test substances that disrupt target gene product-binding partner interaction can be identified. Alternatively, cancer markers protein can be used as a "bait protein" in a two-hybrid assay or three-hybrid assay (*see, e.g.,* U.S. Pat. No. 5,283,317; Zervos et al., Cell 72:223-232 [1993]; Madura et al., J. Biol. Chem. 268.12046-12054 [1993]; Bartel et al., Biotechniques 14:920-924 [1993]; Iwabuchi et al., Oncogene 8:1693-1696 [1993]; and Brent W0 94/10300 ), to identify other proteins, that bind to or interact with cancer markers ("cancer marker-binding proteins" or "cancer marker-bp") and are involved in cancer marker activity. Such cancer marker-bps can be activators or inhibitors of signals by the cancer marker proteins or targets as, for example, downstream elements of a cancer markers-mediated signaling pathway.

Modulators of cancer markers expression can also be identified. For example, a cell or cell free mixture is contacted with a candidate compound and the expression of cancer marker mRNA or protein evaluated relative to the level of expression of cancer marker mRNA or protein in the absence of the candidate compound. When expression of cancer marker mRNA or protein is greater in the presence of the candidate compound than in its absence, the candidate compound is identified as a stimulator of cancer marker mRNA or protein expression. Alternatively, when expression of cancer marker mRNA or protein is less (i.e., statistically significantly less) in the presence of the candidate compound than in its absence, the candidate compound is identified as an inhibitor of cancer marker mRNA or protein expression. The level of cancer markers mRNA or protein expression can be determined by methods described herein for detecting cancer markers mRNA or protein.

A modulating agent can be identified using a cell-based or a cell free assay, and the ability of the agent to modulate the activity of a cancer markers protein can be confirmed *in vivo, e.g.,* in an animal such as an animal model for a disease (*e.g.*, an animal with prostate cancer or metastatic prostate cancer; or an animal harboring a xenograft of a prostate cancer from an animal (*e.g*., human) or cells from a cancer resulting from metastasis of a prostate cancer (*e.g*., to a lymph node, bone, or liver), or cells from a prostate cancer cell line.

Disclosed are novel agents identified by the above-described screening assays (*See e.g.,* below description of cancer therapies). An agent identified as described herein (e.g., a cancer marker modulating agent, an antisense cancer marker nucleic acid molecule, a siRNA molecule, a cancer marker specific antibody, or a cancer marker-binding partner) may be used in an appropriate animal model (such as those described herein) to determine the efficacy, toxicity, side effects, or mechanism of action, of treatment with such an agent. Furthermore, novel agents identified by the above-described screening assays can be, *e.g*., used for treatments as described herein.

### VII. Transgenic Animals

Disclosed is the generation of transgenic animals comprising an exogenous cancer marker gene (*e.g.*, AGTR1 or LBP) or mutants and variants thereof (*e.g*., truncations or single nucleotide polymorphisms). The transgenic animal may display an altered phenotype (*e.g.*, increased or decreased presence of markers) as compared to wild-type animals. Methods for analyzing the presence or absence of such phenotypes include but are not limited to, those disclosed herein. The transgenic animals may further display an increased or decreased growth of tumors or evidence of cancer.

The transgenic animals find use in drug (*e.g.*, cancer therapy) screens. Test compounds (*e.g.*, a drug that is suspected of being useful to treat cancer) and control compounds (*e.g.*, a placebo) are administered to the transgenic animals and the control animals and the effects evaluated.

The transgenic animals can be generated via a variety of methods. In some embodiments, Embryonal cells at various developmental stages may be used to introduce transgenes for the production of transgenic animals. Different methods are used depending on the stage of development of the embryonal cell. The zygote is the best target for micro-injection. In the mouse, the male pronucleus reaches the size of approximately 20 micrometers in diameter that allows reproducible injection of 1-2 picoliters (pl) of DNA solution. The use of zygotes as a target for gene transfer has a major advantage in that in most cases the injected DNA will be incorporated into the host genome before the first cleavage (Brinster et al., Proc. Natl. Acad. Sci. USA 82:4438-4442 [1985]). As a consequence, all cells of the transgenic non-human animal will carry the incorporated transgene. This will in general also be reflected in the efficient transmission of the transgene to offspring of the founder since 50% of the germ cells will harbor the transgene. U.S. Pat. No. 4,873,191 describes a method for the micro-injection of zygotes

Retroviral infection may be used to introduce transgenes into a non-human animal. The retroviral vector may be utilized to transfect oocytes by injecting the retroviral vector into the perivitelline space of the oocyte (U.S. Pat. No. 6,080,912. The developing non-human embryo can be cultured *in vitro* to the blastocyst stage. During this time, the blastomeres can be targets for retroviral infection (Janenich, Proc. Natl. Acad. Sci. USA 73:1260 [1976]). Efficient infection of the blastomeres is obtained by enzymatic treatment to remove the zona pellucida (Hogan et al., in Manipulating the Mouse Embryo, Cold Spring Harbor Laboratory Press, Cold Spring Harbor, N.Y. [1986]). The viral vector system used to introduce the transgene is typically a replication-defective retrovirus carrying the transgene (Jahner et al., Proc. Natl. Acad Sci. USA 82:6927 [1985]). Transfection is easily and efficiently obtained by culturing the blastomeres on a monolayer of virus-producing cells (Stewart, et al., EMBO J., 6:383 [1987]). Alternatively, infection can be performed at a later stage. Virus or virus-producing cells can be injected into the blastocoele (Jahner et al., Nature 298:623 [1982]). Most of the founders will be mosaic for the transgene since incorporation occurs only in a subset of cells that form the transgenic animal. Further, the founder may contain various retroviral insertions of the transgene at different positions in the genome that generally will segregate in the offspring. In addition, it is also possible to introduce transgenes into the germline, albeit with low efficiency, by intrauterine retroviral infection of the midgestation embryo (Jahner *et al., supra* [1982]). Additional means of using retroviruses or retroviral vectors to create transgenic animals known to the art involve the micro-injection of retroviral particles or mitomycin C-treated cells producing retrovirus into the perivitelline space of fertilized eggs or early embryos (PCT International Application WO 90/08832 [1990], and Haskell and Bowen, Mol. Reprod. Dev., 40:386 [1995]).

The transgene may be introduced into embryonic stem cells and the transfected stem cells are utilized to form an embryo. ES cells are obtained by culturing pre-implantation embryos *in vitro* under appropriate conditions (Evans et al., Nature 292:154 [1981]; Bradley et al., Nature 309:255 [1984]; Gossler et al., Proc. Acad. Sci. USA 83:9065 [1986]; and Robertson et al., Nature 322:445 [1986]). Transgenes can be efficiently introduced into the ES cells by DNA transfection by a variety of methods known to the art including calcium phosphate co-precipitation, protoplast or spheroplast fusion, lipofection and DEAE-dextran-mediated transfection. Transgenes may also be introduced into ES cells by retrovirus-mediated transduction or by micro-injection. Such transfected ES cells can thereafter colonize an embryo following their introduction into the blastocoel of a blastocyst-stage embryo and contribute to the germ line of the resulting chimeric animal (for review, See, Jaenisch, Science 240:1468 [1988]). Prior to the introduction of transfected ES cells into the blastocoel, the transfected ES cells may be subjected to various selection protocols to enrich for ES cells which have integrated the transgene assuming that the transgene provides a means for such selection. Alternatively, the polymerase chain reaction may be used to screen for ES cells that have integrated the transgene. This technique obviates the need for growth of the transfected ES cells under appropriate selective conditions prior to transfer into the blastocoel.

Homologous recombination may be utilized to knock-out gene function or create deletion mutants (e.g., truncation mutants). Methods for homologous recombination are described in U.S. Pat. No. 5,614,396.

### EXPERIMENTAL

The following examples are provided in order to demonstrate and further illustrate certain preferred embodiments and aspects of the present invention .

### Example 1

### AGTR1 Expression in Breast Cancer

### A. Materials and Methods

### Cancer Outlier Profile Analysis (COPA)

COPA analysis was performed on 12 breast cancer gene expression data sets in Oncomine 3.0 as described (Tomlins et al., Science 310, 644 [2005]). COPA has three steps. First, gene expression values are median-centered, setting each gene's median expression value to zero. Second, the median absolute deviation (MAD) is calculated and scaled to I by dividing each gene expression value by its MAD. Median and MAD were used for transformation as opposed to mean and standard deviation so that outlier expression values do not unduly influence the distribution estimates, and are thus preserved postnormalization. Third, the 75th, 90th, and 95th percentiles of the transformed expression values are tabulated for each gene and then genes are rank-ordered by their percentile scores, providing a prioritized list of outlier profiles (Fig. 5). Genes scoring in the top 50 outliers at any of the three percentile cutoffs were called outliers.

### Tissue Micoarrays

Breast tissue samples were obtained from the Surgical Pathology files at the University of Michigan with Institutional Review Board (IRB) approval. Three hundred and eleven cases of invasive breast cancer were used to construct tissue microarrays using a manual arrayer as described previously (Witkiewicz et al., Cancer Epidemiol Biomarkers Prev 14, 1418 [2005]). Each tumor was sampled in triplicate to account for tumor heterogeneity.

### Fluorescence in situ hybridization (FISH)

Four microns thick tissue microarray sections were used for interphase fluorescence in situ hybridization (FISH). Deparaffinized tissue was treated with 0.2 mol/L HCl for 10 minutes, 2x SSC for 10 minutes at 80°C and digested with Proteinase K (Invitrogen) for 10 minutes. The tissues and BAC probes were codenatured for 5 minutes at 94°C and hybridized overnight at 37°C. Post-hybridization washing was with 2x SSC with 0.1% Tween 20 for 5 minutes, and fluorescent detection was done using anti-digoxigenin conjugated to fluorescein (Roche Applied Science, Indianapolis, IN) and streptavidin conjugated to Alexa Fluor 594 (Invitrogen). Slides were counterstained and mounted in ProLong Gold Antifade Reagent with 4V, 6- diamidino-2-phenylindole (Invitrogen).

Slides were examined using an Axioplan ImagingZ1 microscope (Carl Zeiss) and imaged with a CCD camera using the ISIS software system in Metafer image analysis system (MetaSystems, Altlussheim, Germany). FISH signals were scored manually (100x oil immersion) by a pathologist and enumerated in morphologically intact and nonoverlapping nuclei. Amplification was defined as a locus number to control copy number of 1.5 or greater (Prentice et al., Oncogene 24, 7281 [2005]).

All BACs were obtained from the BACPAC Resource Center (Oakland, CA), and probe locations were verified by hybridization to metaphase spreads of normal peripheral lymphocytes. For detection of locus and control signal numbers, RP11-505J9 (mapping to AGTR1 locus on 3q24) and RP11-449F7 (3q control) were used, respectively. BAC DNA was isolated using a QIAFilter Maxi Prep kit (Qiagen, Valencia, CA), and probes were synthesized using digoxigenin- or biotin-nick translation mixes (Roche Applied Science).

### Cell Invasion Assay

Breast cell lines BT-549, Hs 579, MCF7, H16N2 and prostate carcinoma line DU 145 were grown in 100mm tissue culture plates overnight, then transferred to serum free medium. Losartan (Merck, Whitehouse Station, NJ) was added 30 minutes prior to angiotensin II (American Peptide Company, Sunnyvale, CA) treatment. Cell invasion was evaluated using 24-well Matrigel invasion chambers (Becton Dickinson, Franklin Lakes, NJ). Cells were trypsinized and seeded at equal numbers onto the basement membrane matrix present in the insert of a 24 well culture plate. Fetal bovine serum was added to the lower chamber acting as a chemoattractant. After 48 hours of additional incubation, the non-invading cells and EC matrix were removed gently with a cotton swab. The cells that had invaded were present on the lower side of the chamber and were stained, air-dried and photographed. The invaded cells were counted under the microscope assessing six random fields per experiment. The numbers of cells were averaged and standard deviations were calculated. To assess relative change in invasion, the ratio of cell invasion with AT alone treatment was divided by the cell invasion at baseline. To assess % reduction in invasion, the cell invasion with AT + losartan treatment (2 µM) was subtracted from the AT alone treatment and then divided by AT alone treatment.

### Quantitative PCR (QPCR)

Quantitative PCR (QPCR) was performed using SYBR Green dye on an Applied Biosystems 7300 Real Time PCR system (Applied Biosystems, Foster City, CA) essentially as described (Tomlins et al., *supra*). Briefly, total RNA was isolated from three 10-micron sections from each formalin fixed paraffin embedded (FFPE) tissue specimen using a MasterPure RNA Purification Kit (Epicentre, Madison, WI) according to the manufacturer's instructions and treated with DNAse I. Total RNA was isolated from cell lines using Trizol (Invitrogen, Carlsbad, CA). RNA was quantified using a ND-1000 spectrophotometer (Nanodrop Technologies, Wilmington, DE) and 1-5 µg of total RNA was reverse transcribed into cDNA using SuperScript II (Invitrogen) in the presence of random primers. All QPCR reactions were performed in duplicate with SYBR Green Master Mix (Applied Biosystems) and 25 ng of both the forward and reverse primer using the manufacturer's recommended thermocycling conditions. For each experiment, threshold levels were set during the exponential phase of the QPCR reaction using Sequence Detection Software version 1.2.2 (Applied Biosystems). For experiments using RNA isolated from FFPE tissues, the amount of AGTR1 relative to the housekeeping gene GAPDH for each sample was determined using the comparative threshold cycle (Ct) method (Applied Biosystems User Bulletin #2). For experiments using RNA from cell lines, the amount of AGTR1 relative to the average of the housekeeping genes GAPDH, B2M, and HMBS was determined for each sample. For all experiments, the relative amount of AGTR1 for each sample was calibrated to the median amount from all samples in the experiment. All oligonucleotide primers were synthesized by Integrated DNA Technologies (Coralville, 1A). B2M (Hudziak et al., Mol Cell Biol 9, 1165 [1989]), GAPDH and HMBS (Piccart-Gebhart et al., N Engl J Med 353, 1659 [2005]) primers were as described. Sequences for AGTR1 I are as follows:
AGTR1_f-GCTTTCCTACCGCCCCTCAGA (SEQ ID NO:1)
AGTR1_r-TTTCGAACATGTCACTCAACCTCAA (SEQ ID NO:2)
Approximately equal efficiencies of the primers were confirmed using serial dilutions of pooled breast cancer RNA in order to use the comparative Ct method. All reactions were subjected to melt curve analysis.

### B. Results

A central aim in cancer research is to identify genetic alterations that play a casual role in the pathogenesis of cancer, thereby providing an opportunity to develop therapies that directly target the alterations. In breast cancer research, this strategy has been successfully realized with the study of ERBB2, which is amplified and over-expressed in 25-30% of breast tumors (King et al., Science 229, 974 [1985]; Slamon et al., Science 235, 177 [1987]), directly contributing to tumorigenesis (Di Fiore et al., Science 237, 178 [1987]; Hudziak et al., Mol Cell Biol 9, 1165 [1989]). Targeting this genetic lesion with trastazumab, a humanized monoclonal antibody directed against ERBB2, has significant clinical benefit in breast cancer management (Piccart-Gebhart et al., N Engl J Med 353, 1659 [2005]; Romond et al., N Engl J Med 353, 1673 [2005]; Slamon et al., N Engl J Med 344, 783 [2001]).

A data mining strategy that searches for genes with very high over-expression in a subset of tumor samples was utilized. When applied to the Oncomine database (Rhodes et al., Proc Natl Acad Sci U S A 101, 9309 [2004]; Rhodes et al., Neoplasia 6, 1 [2004]), the methodology, termed Cancer Outlier Profile Analysis (COPA), correctly identified several known oncogenes, including PBX1 in leukemia and CCND1 in multiple myeloma (Tomlins et al., Science 310, 644 [2005]). In addition, COPA nominated ERG and ETV1 as candidate oncogenes in prostate cancer prompting the discovery of recurrent chromosomal rearrangements involving ERG or ETV1 and the androgen-regulated gene, TMPRSS2 (Tomlins et al., [2005], supra).

Several of the oncogenes correctly identified by COPA had consistent outlier expression profiles in multiple independent gene expression profiling datasets. Thus, in this study, the COPA methodology was combined with cross-study validation to identify genes with strong outlier profiles in multiple independent breast cancer gene expression datasets.

### Outlier Analysis of Breast Cancer Gene Expression Data

Novel oncogenes in breast cancer were identified by searching for genes with strong outlier profiles in multiple independent gene expression profiling datasets. Twelve datasets (Gruvberger et al., Cancer Res 61, 5979 [2001]; Huang et al., Lancet 361, 1590 [2003]; Ma et al., Proc Natl Acad Sci U S A 100, 5974 [2003]; Perou et al., Nature 406, 747 [2000]; Sorlie et al., Proc Natl Acad Sci U S A 98, 10869 [2001]; Sorlie et al., Proc Natl Acad Sci U S A 100, 8418 [2003]; Sotiriou et al., Proc Natl Acad Sci U S A 100, 10393 [2003]; van de Vijver et al., N Engl J Med 347, 1999 [2002]; van 't Veer et al., Nature 415, 530 [2002]; Wang et al., Lancet 365, 671 [2005]; West et al., Proc Natl Acad Sci U S A 98, 11462 [2001]; Zhao et al., Mol Biol Cell 15, 2523 [2004]) with more than 25 breast cancer cases each were selected from the Oncomine database (Rhodes et al., Neoplasia 6, 1 [2004]) for analysis by the COPA methodology (Fig. 5). In each dataset, genes were rank-ordered by their COPA scores at three percentile cutoffs: 75th, 90th and 95th. For each dataset, outlier genes were defined as those that ranked in the top 50 COPA scores at any one of the percentile cutoffs. Seventeen genes were called outliers in at least 4 of the 12 datasets (Table 1). ERBB2 was called an outlier in 6 of 12 datasets, as were 6 other genes that localize near ERBB2 on chromosome 17q. This is consistent with the previous observation that ERBB2 and genomic neighbors are co-amplified and coexpressed in breast cancer (Bertucci et al., Oncogene 23, 2564 [2004]; Kauraniemi et al., Cancer Res 61, 8235 [2001]). Figure 1 depicts a co-expression heatmap of ERBB2 in the van de Vijver ct al study in which ERBB2 and several genomic neighbors were called outliers and show strong co-expression. None of the other highly scoring outliers showed this pattern of co-expression with genomic neighbors, suggesting that their over-expression is mediated by transcriptional regulation, single gene amplification or chromosomal rearrangement.

AGTR1, the gene encoding angiotensin II receptor type I was investigated further. This receptor is the target of a class of highly-specific and widely prescribed cardiovascular drugs, including losartan (Timmermans, Hypertens Res 22, 147 [1999]). In addition to being a well characterized therapeutic target, AGTR1 has a body of literature supporting its designation as a candidate oncogene. For example, AGTR1 blockade has been shown to reduce proliferation, invasion and metastasis in a variety of systems including mouse models of renal cell carcinoma, prostate cancer and ovarian carcinoma (Timmermans, Hypertens Res 22, 147 [1999]; Miyajima et al., Cancer Res 62, 4176 [2002]; Fujimoto et al., FEBS Lett 495, 197 [2001]; Rivera et al., Br J Cancer 85, 1396 [2001]; Uemura et al., Mol Cancer Ther 2, 1139 [2003]; Suganuma et al., Clin Cancer Res 11, 2686 [2005]). Also, angiotensin signaling through AGTR1 leads to activation of the PI3K and MAPK pathways (Muscella et al., J Cell Physiol 197, 61 [2003]; Amaya et al., Int J Oncol 25, 849 [2004]). AGTR1 was one of eight genes that were called an outlier in 6 of 12 datasets (Table 1). Figure 2 depicts the outlier expression pattern of AGTR1 in two representative breast cancer datasets, one of which included normal breast tissue samples. Data from five additional datasets are presented in Figs. 6, 7.

The first dataset showed AGTR1 to be relatively under-expressed in most tumors relative to normal breast tissue, but markedly over-expressed in a fraction of tumors (Fig. 2A), whereas the second dataset showed that AGTR1 over-expression is confined to a subset of estrogen receptor (ER) positive tumors (Fig. 2C). The outlier profile and association with ER status was recapitulated in other independent datasets (Figs. 6, 7).

Next, the relationship of AGTR1 over-expression and ERBB2 over-expression were investigated. In each of the 7 datasets examined, these two genes showed a mutually exclusive expression pattern. Tumors invariably overexpressed either ERBB2 or AGTR1, but never both (Figs. 2B, D, Figs. 6, 7). It is contemplated that ERBB2 over-expression and AGTR1 over-expression represent alternative pathways in breast cancer pathogenesis. Given the consistent and marked over-expression of AGTR1 in a fraction of breast cancers that are invariably ER+ / ERBB2- and past work demonstrating the functional importance of AGTR1 in tumorigenesis, it was contemplated that AGTR1 over-expression is mediated by recurrent DNA amplifications or translocations.

### Fluorescence in Situ Hybridization on Tissue Microarrays

To test for DNA aberrations at the AGTR1 locus, fluorescence in situ hybridization (FISH) was performed on tissue microarrays. Samples were first tested for chromosomal rearrangements at the AGTR1 locus using a split probe strategy on tissue microarrays, as described previously for ETV1 in prostate cancer (Tomlins et al., [2005], supra). As 5' and 3' AGTR1 probes never demonstrated consistent split signals, samples were next tested for copy number change using a locus and control probe strategy (Fig. 3A). In total, AGTR1 copy number was evaluated in 112 breast carcinoma cases, of which 106 were invasive ductal carcinoma (IDC) and 6 were ductal carcinoma in situ (DCIS). A first pass evaluation identified 16 cases with multiple cells showing more signals from the AGTR1 locus probe relative to control probe. Exact locus to control ratios (L/C) were tabulated for these 16 cases as well as 14 cases with no evidence of copy number change on first pass evaluation. Seven of the 16 'positive' cases had clear evidence of copy number gain (L/C > 1.5), while none of the 'negative' cases had evidence of gain (0.9 < L/C < 1.2).

In summary, definitive copy number gain was observed in 7 of 112 (6.25%) cases, of which 6 of 106 (5.6%) were invasive ductal carcinoma and 1 was ductal carcinoma in situ. Figure 3B depicts representative cases with and without definitive copy number gain.

To test the hypothesis that the observed over-expression is the result of DNA copy number gain, 14 cases with no gain (L/C < 1.2), 3 cases with questionable gain (1.2 < L/C < 1.5) and 4 cases with definitive gain (L/C > 1.5) were selected for expression analysis. Quantitative RT-PCR analysis was performed and AGTR1 expression was standardized to GAPDH expression defining three bins of AGTR1 levels: low (<1), moderate (1 - 2) and high (>2.0). By RT-PCR analysis, a significant concordance between high AGTR1 expression and definitive copy number gain was identified (Fig. 3C, p-value = 0.006). While 3 of 17 cases without definitive copy number gain had high AGTR1 expression, all 4 of 4 cases with definitive copy number gain had high expression. While the L/C cutoff for defining copy number gain was 1.5, all four positive cases considered had L/C values near or exceeding 2.0, suggesting 2-4 additional copies per cell.

### In vitro analysis of AGTR1

Having shown that AGTR1 is over-expressed in 10-20% of breast cancers and amplified in approximately 6% of tumors, experiments were conducted to provide evidence that AGTR1 over-expression serves as an indicator for treatment with an angiotensin receptor blocker (ARB), such as losartan. Using Oncomine, the expression of AGTR1 in cell lines from the NCI-60 panel was assessed. It was found that AGTR1 had relative low expression in MCF-7 breast cancer cells and DU145 prostate cancer cells and relative high expression in BT549 and 578T breast cancer cells. The expression of AGTR1 in these 4 cell lines was confirmed and AGTR1 expression in the immortalized benign breast epithelial cell line, H 16N2 (Table 2) was measured.

Next, the effect of angiotensin II (AT), the ligand for AGTR1, and losartan, a highly specific AGTR1 blocker, on cell invasion were tested. At baseline, H16N2, BT549 and 578T cells had low invasion, whereas MCF-7 cells were slightly invasive and DU145 cells were highly invasive. Treatment with AT had the strongest positive effect on invasion in the AGTR1 over-expressing cancer cell lines BT549 and 578T (34-and 24-fold increase, respectively) (Table 2, Fig. 4). No effect was observed in the immortalized breast epithelial cell line, H16N2, or the prostate cancer cell line, DU 145, while a moderate effect was observed in the MCF-7 cells (8-fold increase). Losartan significantly reduced AT-mediated invasion in all three cell lines affected by AT (Fig. 4).
At a 2 µM dose, losartan had the strongest effects in the over-expressing lines, reducing AT-mediated invasion in BT549 and 578T cells by 72% and 68%, respectively (Table 2). In summary, AT led to a marked increase in cell invasion in two breast cancer cell lines that over-expressed AGTR1, had a moderate effect in one cancer cell line with low expression, no effect in another cancer cell line with low expression and no effect in an immortalized epithelial cell line with low AGTR1 expression (Fig. 4C). The effects of AT were largely reversed by treatment with losartan, a widely prescribed AGTR1 blocker, indicating that AGTR1 levels may be an indicator for losartan treatment in breast cancer.

In conclusion, experiments showed that the angiotensin receptor, which functions to mediate the vasopressive effects of angiotensin, is consistently one of the most highly overexpressed genes in 10-20% of breast tumors. AGTR1 always displayed high over-expression in estrogen receptor positive, ERBB2-negative tumors. Based on the mutually exclusive expression pattern with ERBB2 and the overlapping downstream pathways affected by AGTR1 and ERBB2, namely the MAPK pathway, it is contemplated that AGTR1 activation and ERBB2 activation represent functionally related events. Similar to ERBB2, AGTR1 is subject to DNA copy number gain. Although amplification did not account for all of the observed overexpression, the demonstration of recurrent chromosomal aberrations in cases that overexpress AGTR1 confirms that AGTR1 amplification and over-expression represent a clonally selected aberration. The recurrent amplifications indicate that AGTR1 is a breast cancer oncogene. Thus, AGTR1 blockers may be used in conjunction with standard chemotherapy for breast cancer patients with AGTR1-over-expressing tumors in both the adjuvant setting and the setting of metastatic disease.

**Table 1. Meta-COPA analysis of 12 breast cancer gene expression profiling datasets in Oncomine.**

| **# Studies** | **Avg.Rank** | **Gene** | **Chr.** |
|---|---|---|---|
| | | | |
| 7 | 11.14 | LBP | 20q |
| 7 | 14.71 | STARD3 | **17q** |
| 7 | 21.86 | FABP7 | 6q |
| 6 | 8.67 | PPARBP | **17q** |
| 6 | 14.83 | CYP2A6 | 19q |
| 6 | 17.00 | AGTR1 | 3q |
| 6 | 24.00 | **ERBB2** | **17q** |
| 5 | 7.40 | GRB7 | **17q** |
| 5 | 13.00 | GRIA2 | 4q |
| 5 | 16.60 | GABRP | 5q |
| 5 | 17.40 | HOXB5 | **17q** |
| 4 | 10.75 | S100A8 | 1q |
| 4 | 11.50 | COL2A1 | 12q |
| 4 | 17.00 | NAT1 | 8p |
| 4 | 17.25 | PSMD3 | **17q** |
| 4 | 28.50 | THRAP4 | **17q** |
| 4 | 30.00 | HOXB6 | **17q** |

**Table 2. AGTR1 expression measurement and invasion assays of cancer cell lines.**

| | | Invasion | | | | | |
|---|---|---|---|---|---|---|---|
| | AGTR1 | Untr. | AT | AT + Lo (1uM) | AT + Lo (2uM) | AT Fold Incr. | Lo (2uM) % Red. |
| BT549 | 54.44 | 2.33 | 80.33 | 55.50 | 22.17 | 34.43 | 72.4% |
| 578T | 260.85 | 2.67 | 63.33 | 32.17 | 20.33 | 23.75 | 67.9% |
| MCF7 | 0.59 | 9.00 | 71.67 | 37.50 | 31.00 | 7.96 | 56.7% |
| H16N2 | 0.88 | 0.67 | 1.00 | 1.17 | 0.83 | 1.50 | 16.7% |
| DU145 | 0.42 | 140.50 | 149.17 | 156.33 | 97.00 | 1.06 | 35.0% |

### Example 2

### LBP Expression in Breast Cancer

A combination of COPA and a meta-analysis strategy identified a small number of genes with very high over-expression in a fraction of breast cancer cases across several independent patient cohorts. In addition to identifying ERBB2, an established breast cancer oncogene and therapeutic target, the analysis also prioritized AGTR1, the angiotensin receptor. AGTR1 was found to be amplified in a subset of over-expressing cases and in vitro studies showed that AGTR1 over-expressing cell lines became invasive with angiotensin treatment, which was attenuated by losartan treatment (Example above).

This example examines LBP, the lipopolysaccharide binding protein, which was the highest ranked gene by the COPA meta-analysis, displaying the most consistent and most marked over-expression in a fraction of tumors across multiple independent patient cohorts (Table 3). LBP was one of only three genes called an outlier in 7 of 12 datasets and had the best average COPA rank (∼11). LBP mediates anti-apoptotic signaling through CD14 and NF-kB activation (Fukuda et al., Invest Ophthalmol Vis Sci 46, 3095 (2005); Guha et al., Cell Signal 13, 85 (2001)) and studies have begun to characterize the potential of LBP as a therapeutic target for bacteremia (Roy et al., J Immunol 167, 2759 (2001)). While the expression and activity of LBP have been studied almost exclusively in white blood cells with respect to the acute phase response, LBP has been reported to be expressed in mouse mammary tissue undergoing involution (Uehara et al., Oncol Rep 15, 903 (2006)).

Figure 8 depicts the outlier expression pattern of LBP in 6 breast cancer datasets. One dataset included normal breast tissue samples and showed that LBP is highly overexpressed relative to normal tissue expression (Fig. 8A). Several other datasets profiling only cancers demonstrated that LBP over-expression is more common in estrogen receptor negative tumors (25-40%) than in ER positive tumors (5-10%) (Fig. 8B-D). Another study showed that LBP over-expression occurs in apocrine-like and basal-like tumors but not in luminal-like tumors (Fig. 8E). Lastly, another study, which examined only ER positive tumors and characterized gene expression profiles based on response to tamoxifen therapy, demonstrated that LBP over-expression in ER+ tumors occurred in patients with recurrence following tamoxifen treatment but not in patients with no recurrence. Given the consistent and marked over-expression of LBP in a fraction of breast cancers and past work demonstrating DNA-level amplifications of genes such as ERBB2 and AGTR1 with similar expression profiles, it was contemplated that cases with marked over-expression of LBP may have underlying DNA amplifications.

To test for DNA aberrations at the LBP locus, fluorescence in situ hybridization (FISH) was performed. First, the expression of LBP was examined in 17 frozen breast carcinoma cases by quantitative RT-PCR (Fig. 9A). LBP was not detectable in 11 cases and was expressed at low levels relative to GAPDH in the remainder of cases, although LBP / GAPDH varied from 1/6000 to 1/38, thus the case with highest LBP had 150X more message than did the lowest detectable case. FISH analysis was performed on the two cases with highest LBP expression (1:38 and 1:42). In one case a high level DNA copy number gain was observed (Fig. 9B), indicating that LBP amplification and overexpression may be a clonally selected aberration.

**Table 3. Meta-COPA analysis of 12 breast cancer gene expression profiling datasets in Oncomine. Genes were ranked by the number datasets in which they scored in the top 50 outliers (ranked by COPA) at any of the three pre-defined percentile cutoffs (75th, 90th, 95th). Genes that localize to 17q adjacent to ERBB2 are denoted in bold.**

| # Studies | Avg. Rank | Gene | Chr |
|---|---|---|---|
| 7 | 11.14 | LBP | 20q |
| 7 | 14.71 | STARD3 | 17q |
| 7 | 21.86 | FASP7 | 6q |
| 6 | 8.67 | PPARBP | 17q |
| 6 | 14.83 | CYP2A6 | 19q |
| 5 | 17.00 | LBP | 3q |
| 6 | 24.00 | ERBB2 | 17q |
| 5 | 7.40 | GRB7 | 17q |
| 5 | 13.00 | GRIA2 | 4q |
| 5 | 16.60 | GABRP | 5q |
| 5 | 17.40 | HOXB5 | 17q |
| 4 | 10.75 | ST100A8 | 1q |
| 4 | 11.50 | COL2A1 | 12q |
| 4 | 17.00 | NAT1 | 8p |
| 4 | 17.25 | PSMD3 | 17q |
| 4 | 28.50 | THRAP4 | 17q |
| 4 | 30.00 | HOXB6 | 17q |

## Claims

1. A method for identifying breast cancer in a subject, comprising:
a) detecting the copy number of angiotensin II receptor type I (AGTR1) genomic DNA in a sample from a subject; and
b) identifying breast cancer in said subject when a gain in copy number > 1.5 of AGTR1 genomic DNA is present in said sample.

2. The method of claim 1, wherein said breast cancer is estrogen receptor positive breast cancer.

3. The method of claim 1, further comprising the step of determining a treatment course of action based on the level of AGTR1 genomic DNA in said sample.

4. The method of claim 1, wherein ERBB2 is not overexpressed in breast cancer samples having AGTR1 overexpression.

5. An AGTR1 inhibitor selected from the group consisting of Losartan, Valsartan, Eprosartan, Candesartan, Irbesartan, Telmisartan and Olmesartan for use in therapy of breast cancer wherein the therapy comprises:
a) detecting the copy number of angiotensin II receptor type I (AGTR1) genomic DNA in a sample from a subject; and
b) treating said subject with one of said AGTR1 inhibitors when a gain in copy number > 1.5 of AGTR1 genomic DNA is present in said sample.

6. The method of claim 5, wherein said sample is post-surgical tissue.

## Patentansprüche

1. Verfahren zur Identifikation von Brustkrebs bei einem Subjekt, umfassend:
a) die Detektion der Anzahl von Kopien an Angiotensin-II-Rezeptortyp I (AGTR1) genomischer DNA in einer Probe von einem Subjekt; und
b) die Identifikation von Brustkrebs bei dem Subjekt, wenn bei dem Subjekt eine Zunahme der Anzahl von Kopien von > 1,5 an AGTR1 genomischer DNA in der Probe vorliegt.

2. Verfahren nach Anspruch 1, wobei der Brustkrebs Östrogen-Rezeptor positiver Brustkrebs ist.

3. Verfahren nach Anspruch 1, des Weiteren umfassend den Schritt der Festlegung der Behandlungsvorgehensweise basierend auf dem Niveau an AGTR1 genomischer DNA in der Probe.

4. Verfahren nach Anspruch 1, wobei in den Brustkrebsproben mit AGTR1-Überexpression ERBB2 nicht überexprimiert ist.

5. AGTR1-Inhibitor ausgewählt aus der Gruppe bestehend aus Losartan, Valsartan, Eprosartan, Candesartan, Irbesartan, Telmisartan und Olmesartan zur Verwendung in der Therapie von Brustkrebs, wobei die Therapie umfasst:
a) die Detektion der Anzahl von Kopien an Angiotensin-II-Rezeptortyp I (AGTR1) genomischer DNA in einer Probe von einem Subjekt; und
b) die Behandlung des Subjekts mit einem der AGTR1-Inhibitoren, wenn in der Probe eine Zunahme der Anzahl von Kopien von > 1,5 an AGTR1 genomischer DNA vorliegt.

6. Verfahren nach Anspruch 5, wobei die Probe postoperatives Gewebe ist.

## Revendications

1. Procédé pour identifier un cancer du sein chez un sujet, qui consiste à :
a) détecter le nombre de copies de l'ADN génomique du récepteur de l'angiotensine II de type I (AGTR1) dans un échantillon d'un sujet ; et
b) identifier un cancer du sein chez ledit sujet lorsqu'une augmentation du nombre de copies > 1,5 de l'ADN génomique d'AGTR1 est présente dans ledit échantillon.

2. Procédé selon la revendication 1, dans lequel ledit cancer du sein est un cancer du sein à récepteurs d'oestrogènes positifs.

3. Procédé selon la revendication 1, comprenant en outre l'étape qui consiste à déterminer un plan d'action thérapeutique basé sur le taux d'ADN génomique d'AGTR1 dans ledit échantillon.

4. Procédé selon la revendication 1, dans lequel ERBB2 n'est pas surexprimé dans des échantillons de cancer du sein présentant une surexpression d'AGTR1.

5. Inhibiteur d'AGTR1 sélectionné dans le groupe consistant en le losartan, le valsartan, l'éprosartan, le candésartan, l'irbésartan, le telmisartan et l'olmésartan, destiné à être utilisé dans une thérapie du cancer du sein, où la thérapie consiste à :
a) détecter le nombre de copies de l'ADN génomique du récepteur de l'angiotensine II de type I (AGTR1) dans un échantillon d'un sujet ; et
b) traiter ledit sujet avec l'un desdits inhibiteurs d'AGTR1 lorsqu'une augmentation du nombre de copies > 1,5 de l'ADN génomique d'AGTR1 est présente dans ledit échantillon.

6. Procédé selon la revendication 5, dans lequel ledit échantillon est un tissu post-opératoire.
